# EUROPEAN PATENT APPLICATION

(11) **EP 1 854 873 A2**
(43) Date of publication of application: **14.11.2007**
(21) Application number: 07016852.1
(22) Date of filing: 30.01.2001
(51) Int. Cl.: C12M 1/34, C12Q 1/06, G01N 1/30, G01N 33/58

(54) **Microorganism detecting kit, microorganism counting apparatus, and microorganism counting process**

(30) Priority: 31.01.2000 JP 2000021656; 31.01.2000 JP 2000021657; 29.03.2000 JP 2000090372; 11.12.2000 JP 2000375643
(62) Divisional of application: 01901562.7
(71) Applicant: Matsushita Ecology Systems Co., Ltd., Kasugai, Aichi 486-8522 (JP)
(72) Inventor: Shimakita, Tomonori, Komaki-shi Aichi 486-8522 (JP); Tashiro, Yoshikazu, Kasugai-shi Aichi 486-0833 (JP); Nashimoto, Kazuo, Kasugai-shi Aichi 486-0833 (JP)
(74) Representative: Thun, Clemens

(57) **Abstract**

A microorganism counting process is described comprising the steps of bringing 4', 6-diamidino-2-phenylindole dihydrochloride for staining living and dead cells and propidium iodide for staining dead cells at a wavelength different from said coloration into contact with a specimen, and detecting both of living cells and dead cells simultaneously from a difference between wavelengths and difference between amounts of colors developed.

## Description

### TECHNICAL FIELD

The present invention relates to a microorganism detecting kit capable of counting living cells, dead cells and particular microorganisms at one time by the color development of the cells from a specimen with a plurality of types of microorganisms (the concept of which means the inclusion of bacteria and true fungi), i.e., microorganisms such as bacteria and true fungi possibly deposited thereon or incorporated therein, and a microorganism counting apparatus and a microorganism counting process capable of counting the color-developed microorganisms.

### BACKGROUND ART

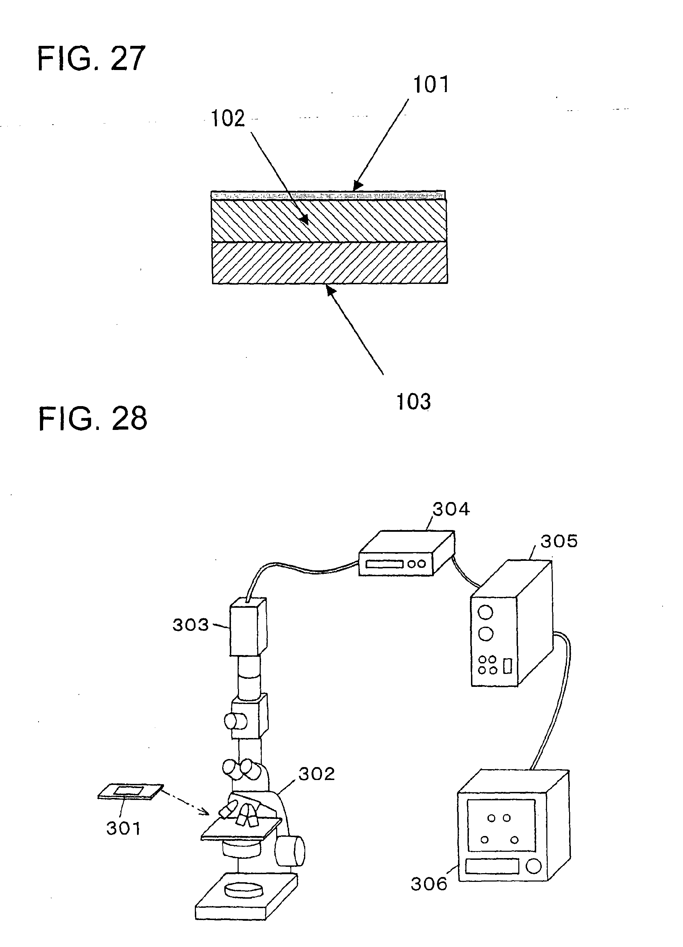

As for such kit for detecting the microorganisms, there are known a self-adhesive sheet as described in Japanese Patent Application Laid-open No.10-70975, and a plate counting process, which is a common process for determining the number of deposited cells. The detail of such detecting kit is shown in Fig.27. The self-adhesive sheet 101 is comprised of a self-adhesive layer 102 and a support 103. Microorganisms in a specimen are brought into a contact with the self-adhesive layer 102 and collected on a surface of the self-adhesive layer 102. An antibody and/or an aqueous solution of a color-developing substrate for microorganisms to be detected is brought into contact with the surface of the self-adhesive layer 102, and the color development of the microorganisms on the surface of the self-adhesive layer 102 is observed visually or by a microscope, thereby confirming the presence or absence of the microorganisms and the number of the microorganisms.

There is also known a deposited- bacterium measuring kit (not shown) based on a plate counting process as one of culturing processes, which are common processes for determining the number of bacteria or fungi. The deposited-baoterium measuring kit is adapted to judge the presence or absence of microorganisms and count the number of microorganisms by bringing the surface of a culture medium into direct contact with a specimen, collecting microorganisms on the surface of the culture medium, culturing the microorganism deposited to the surface of the culture medium as they are, and observing them visually or by a microscope or the like.

Such conventional microorganism detecting kit requires steps of bringing the self-adhesive sheet into contact with the specimen, collecting the microorganisms and then applying a reagent. For this reason, a time and a technique are required for an examining operation. An immune process for detecting particular microorganisms using an antibody has a high sensitiveness, because the antibody is bonded specifically with an -antigen. In the immune process, however, the use of the antibody is essentially required and for this reason, there are problems in respect of the nature itself of the antibody such as the production and management of the antibody and an immune reaction detecting means. The immune process is not capable of judging the life or death of cells. In addition, if the dyeing of the antibody is used, the self-adhesive layer is dyed to a dyed color and after the dyeing, colors other than the specimen deposited to the self-adhesive layer are required to be removed. Further, the antibody and the color-developing substrate are used, and the entire area to be detected is dyed and for this reason, particular microorganisms can be detected, but cannot be detected simultaneously with the common bacteria.

There are such conventionally known apparatus for counting microorganisms, which is designed to apply exciting light directly to a sample containing microorganisms, acquire fluorescent rays emitted from the fluorescent substances produced in the microorganisms as image data, measure a concentration of the fluorescent substances in the microorganisms and measure the activity of the microorganisms from the addition of the intensity of the fluorescent ray, as described in Japanese Patent Application Laid-open No.5-111394. The detail of such microorganism counting process is shown in Fig.28. A suspension of methanogens germs is placed on a preparation 301, and exciting light 302 is applied to the suspension. Fluorescent rays generated are recognized using ahigh-sensitiveness television camera 303 and VTR 304 and stored. The intensity of the intended fluorescent ray is detected from an image picture projected on a monitor television 306 using a picture-processing device 305.

Such conventional microorganism counting process cannot be suited for microorganisms producing no fluorescent substance. In addition, in the conventional microorganism counting process, microorganisms incapable of producing a fluorescent substance because of a weaker activity are not detected in some oases, even if they are target microorganisms. The activity or number of the microorganisms cannot be necessarily detected. The microorganism counting apparatus is used in food factories in many oases. In such case, it is an important factor for the management how many are all microorganisms present rather than particular microorganisms. Therefore, for as the standard of living microorganisms on a fresh vegetable, the number of common bacteria is 1,000,000/ml or less. According to the introduction of a latest HACCP, not only the examination of the number of living germs on a food itself but also the examination in a factory itself and in an operating procedure, e.g., the examination of living germs on a wall, a floor surface, cooking devices such as a chopping board and a kitchen knife have been conducted, and the management of a food itself and the management of environments are important. However, the conventional technique is complicated and requires a specialized knowledge and thus, it is not true that anybody can measure microorganism easily in a field using the conventional technique. Even in respect of the apparatus, exciting light is applied to fluorescent substances produced by microorganisms, and fluorescent rays emitted from the fluorescent substances are analyzed by processing of a picture. For this reason, a technique for enlarging the picture at high magnifications as in a microscope is required.

The common culturing process for detecting the deposited germs suffers from a problem that a lot of time is taken for culturing deposited microorganisms. Further, some of types of microorganisms may be not bred on a culture medium having the microorganisms deposited thereon in some oases, which may cause an underestimation. Furthermore, the culturing process detects only living germs and for this reason, the number and the life or death of microorganisms existing in the environments cannot be necessarily confirmed by the culturing process.

There are such conventionally known microorganism counting processes and apparatus, in which an added compound is permeated into microorganism bodies and then decomposed by an enzyme reaction in the microorganism bodies, and microorganisms emitting fluorescent rays are detected, as described in Japanese Patent Application Laid-open No. 10-323197. These microorganisms are recognized as living microorganisms. In addition, the numbers of living and dead microorganisms are grasped by using a reagent for dyeing the dead cells in combination with an above-described reagent. There are other conventionally known techniques, in which particular microorganisms are detected in a luminescent manner using an antibody, as described in Japanese Patent Application Laid-open No.7-140148, and in which the number of particular microorganisms and the numbers of living and dead microorganisms are grasped using a reagent for dyeing the microorganisms non-specifically in combination with an antibody.

In a food sanitation field or the like, it is desired to detect particular microorganisms promptly. Coliform baoiteria and particular E.coli are measured as the particular microorganisms, and depending on the type of food, particular microorganisms such as staphylococcus are measured. However, in a situation in which particular microorganisms coexist together with common bacteria or in a situation in which impurities exist, it is required that the particular microorganisms are separated or separated and cultured using a separating means, or the impurities are removed. The common bacteria and the particular microorganisms cannot be counted simultaneously.

In such conventional microorganism counting process and apparatus, a process for discriminating microorganisms promptly described in Japanese Patent Application Laid-open No. 10-323197 uses a fluorescent reagent comprising fluorescein or the derivative thereof and a fluorescent dye comprising propidium iodide under limited conditions. However, the fluorescent reagent comprising fluorescein or the derivative thereof is subject to a course in which it is decomposed by esterase produced by microorganisms to emit rays, and hence, the fluorescent reagent largely depends on the activity of the enzyme. Namely, an enzyme activation temperature or the substrate specificity varied depending on the type of microorganisms is exemplified as an error influence factor. Therefore, in environments in which the same type of microorganisms only exists, a high-sensitiveness measurement is expected, but there is a high possibility that an underestimation or an overestimation may be caused in actual service environments. The esterase may be produced even outside microorganism cells. In such a case, the microorganisms are integrated with a background and for this reason, there is a possibility that an accurate detection cannot be achieved. Therefore, there is a problem that even if the fluorescent reagent is used in combination with the propidium iodide, or the double dyeing is carried out, the numbers of living and dead microorganisms cannot be necessarily grasped.

On the other hand, the microorganism counting process described in Japanese Patent Application Laid-open No.7-140148 employs an antibody to detect specific microorganisms and a dyeing reagent to be non-specifically bonded to microorganisms. In this process, it is requisite to use the antibody, but the antibody is high in sensitiveness, because it is bonded specifically, and on the other hand, the life and the death cannot be discriminated. Further, a process using this technique cannot discriminate the life and death of microorganisms dyed by the non-specifically dyeing reagent contained in a specimen.

It is also desired to measure particular microorganisms and common bacteria simultaneously and promptly.

The present invention solves such a conventional subject, and it is an object of the present invention to provide a microorganism detecting kit, wherein a type or a plurality of types of staining or coloring compounds are incorporated into a specimen contact portion, whereby the collection and detection of cells can be conducted simultaneously to reduce the reagent applying step, and microorganisms can be detected promptly. It is also an object of the present invention to provide a microorganism detecting kit, wherein to detect particular microorganisms, it is unnecessary to remove microorganisms other than the particular microorganisms and proteins which are impurities, and it is also unnecessary to separate impurities and microorganisms other than the subject microorganisms incorporated in a specimen, and the particular microorganisms can be detected simultaneously and promptly.

It is a further object of the present invention to provide a microorganism detecting kit, wherein a detecting-reagent containing section having a staining or coloring compound incorporated therein is provided in addition to a specimen contact portion, whereby microorganisms can be detected by adding a detecting reagent to the specimen contact portion without being deposited directly to a specimen, and the microorganisms can be detected safely, because an operator cannot touch the specimen contact portion and the detecting-reagent containing section directly, leading to an enhancement in microorganism detecting operability; and particular microorganisms can be detected simultaneously.

It is a yet further object of the present invention to provide a microorganism detecting kit, wherein exciting light and fluorescent rays emitted by the exciting light and having a particular wavelength can be controlled by carrying out the staining by fluorescent rays, and cells to be detected can be discriminated easily and simply.

Further, it is an object of the present invention to provide a microorganism detecting kit, wherein the numbers of living and dead cells or the number of dead cells can be counted at a single-cell level by using a compound suitable to be bonded to nucleic acid in the living and dead cells or the dead cells.

Yet further, it is an object of the present invention to provide a microorganism detecting kit, wherein the number of only living microorganisms can be counted by incorporating a compound for staining living cells by the reaction with a substance derived from the microorganisms.

Yet further, it is an object of the present invention to provide a microorganism detecting kit, wherein the judgment can be achieved with a good sensitiveness by ensuring that the substance derived from the microorganisms is an enzyme protein.

Yet further, it is an object of the present invention to provide a microorganism detecting kit, wherein the presence or absence of particular microorganisms can be judged with a higher sensitiveness by ensuring that the substance derived from the particular microorganisms is an enzyme protein, and further, the life or death of the microorganisms can be judged from the reactivity by the measurement with the passage of time.

Yet further, it is an object of the present invention to provide a microorganism detecting kit, wherein by separating compounds for developing colors by the reaction from one another, the color development provided by the compounds can be visually confirmed.

Yet further, it is an object of the present invention to provide a microorganism detecting kit, wherein it is possible to facilitate the storage of the compound by supporting the compounds on a carrier, and the detecting sensitiveness can be increased by increasing the area of contact with a specimen.

Yet further, it is an object of the present invention to provide a microorganism detecting kit, wherein it is possible to facilitate the storage of the compound by supporting the compounds on a carrier, and the detecting sensitiveness can be increased by increasing the area of contact with a specimen, and wherein by separating the compound-supporting carriers developing the color by the reaction from one another, the color development provided by the compounds can be visually confirmed.

Yet further, it is an object of the present invention to provide a microorganism detecting kit, wherein the need for the scanning on a specimen contact means can be eliminated by setting the size of at least a specimen contact portion of the specimen contact means to be equal to the size of a light-receiving means of an optical microorganism counting apparatus.

Yet further, it is an object of the present invention to provide a microorganism detecting kit, wherein the reactivity of one or more compounds with microorganisms contained in a specimen can be increased by supporting the one or more compounds on a surface of a specimen contact portion.

Yet further, it is an object of the present invention to provide a microorganism detecting kit, wherein a reduction in activity of collected cells can be prevented by containing a culturing component in a specimen contact means.

Yet further, it is an object of the present invention to provide a microorganism detecting kit, wherein a microorganism collecting effect can be enhanced by providing a specimen contact portion with a self-adherent property, whereby microorganisms deposited to a specimen can be counted precisely.

Yet further, it is an object of the present invention to provide a microorganism detecting kit, wherein a self-adhesive layer can be pressed in correspondence to an unevenness of a specimen and hence, microorganisms can be collected irrespective of the shape of the specimen and further, it is possible to prevent an operator from touching the specimen or a specimen contact portion after deposition of the microorganism to the specimen.

Yet further, it is an object of the present invention to provide a microorganism detecting kit, wherein a special light-separating means is not required by providing the microorganism detecting kit with a light-separating function, and the confirmation of the color development can be simplified.

Yet further, it is an object of the present invention to provide a microorganism detecting kit, wherein a special light-separating means is not required by providing a detecting-reagent containing section with a light-separating function; the confirmation of the color development can be simplified, and the switching placement of a means for separating light having another wavelength can be also conducted easily.

Yet further, it is an object of the present invention to provide a microorganism detecting kit, wherein it is possible to accommodate even a liquid specimen by including a preservation reservoir formed so that the liquid specimen is of a given thickness, and an error due to a difference in thickness can be prevented.

Yet further, it is an object of the present invention to provide a microorganism counting apparatus, comprising a specimen contact means in which any one or some of the following compounds are incorporated: a first compound for staining living and dead cells, a second compound for staining dead cells at a wavelength different from the above-desaribed staining, a third compound for staining living cells at a wavelength different from the above-described staining, and at least one or more fourth compounds for staining particular microorganisms at a wavelength different from the above-described staining by the reaction with a substance derived from particular microorganisms; a light source for applying exciting light in a predetermined range of wavelength to a very small given area of the specimen contact means; a light-receiving means for receiving light emitted in a predetermined range of wavelength by virtue of the exciting light; a microorganism judging means adapted to receive the rays emitted by the application of the light from the light source within a preset given time for judging microorganisms, and to determine that microorganisms exist when the amount of light received is in a preset threshold value range; a moving means for moving the very small given area continuously or intermittently; and a means for adding the numbers of microorganisms from signals indicative of the judgment as microorganism from the microorganism judging means.

Yet further, it is an object of the present invention to provide a microorganism counting apparatus, including a detecting-reagent containing section and a specimen contact means, the detecting-reagent containing section containing one or some of the following compounds incorporated therein: a first compound for staining living and dead cells, a second compound for staining dead cells at a wavelength different from the above-described staining, a third compound for staining living cells at a wavelength different from the above-described staining, and at least one or more fourth compounds for staining particular microorganisms at a wavelength different from the above-described staining by the reaction with a substance derived from particular microorganisms; a light source for applying exciting light in a predetermined range of wavelength to a very small given area of the specimen contact means; a light-receiving means for receiving light emitted in a predetermined range of wavelength by virtue of the exciting light; a microorganism judging means adapted to receive the rays emitted by the application of the light from the light source within a preset given time for judging microorganisms, and to determine that microorganisms exist when the amount of light received is in a preset threshold value range; a moving means for moving the very small given area continuously or intermittently; and a means for adding the numbers of microorganisms from signals indicative of the judgment as microorganism from the microorganism judging means.

Yet further, it is an object of the present invention to provide an apparatus capable of counting microorganisms by bringing a surface of the specimen contact means into contact with a microorganism-containing specimen or a specimen to be examined whether or not microorganisms are contained therein, or by bringing a transferring means brought into contact with the specimen into contact with the surface of the specimen contact means to transfer the microorganisms in the specimen to the specimen contact means.

Yet further, it is an object of the present invention to provide an apparatus including the specimen contact means in the form of a preservation reservoir for storing a liquid specimen, so that the liquid specimen stored in the preservation reservoir is of a given thickness.

Yet further, it is an object of the present invention to provide an apparatus in which the staining or coloration of microorganisms is fluorescent.

Yet further, it is an object of the present invention to provide an apparatus including the first compound adapted to develop a color by the reaction with a substance derived from microorganisms.

Yet further, it is an object of the present invention to provide an apparatus including the second compound capable of being bonded to nucleic acid.

Yet further, it is an object of the present invention to provide an apparatus including the third compound capable of being bonded to nucleic acid.

Yet further, it is an object of the present invention to provide an apparatus in which the substance derived from microorganisms, with which the first compound reacts, is an enzyme protein.

Yet further, it is an object of the present invention to provide an apparatus in which the substance derived from particular microorganisms, with which the fourth compound reacts, is an enzyme protein.

Yet further, it is an object of the present invention to provide an apparatus in which the light-receiving means is at least one photoelectric converting element, and a collector lens for collecting the light emitted by the exciting light is mounted in front of the photoelectric converting element.

Yet further, it is an object of the present invention to provide an apparatus in which a plurality of photoelectric converting elements are disposed in a linear arrangement.

Yet further, it is an object of the present invention to provide an apparatus in which the specimen contact means is disk-shaped and movable.

Yet further, it is an object of the present invention to provide an apparatus in which the specimen contact means has a polygonal measuring area and is movable.

Yet further, it is an object of the present invention to provide an apparatus in which a drive means for moving any one or some of the light source, the light-receiving means and the specimen contact means is mounted as a moving means for moving a very small area, so that it or they can be moved in a preset range of speed.

Yet further, it is an object of the present invention to provide an apparatus in which a reflecting plate is mounted as the collector means for collecting the light emitted from the light source and/or the collector means for collecting the light to the light-receiving means.

Yet further, it is an object of the present invention to provide an apparatus in which a light-separating section adapted to transmit only light having a particular wavelength is mounted between the light source and the very small area.

Yet further, it is an object of the present invention to provide an apparatus in which a light-separating section adapted to transmit only light having a particular wavelength is mounted between the very small area and the light-receiving means.

Yet further, it is an object of the present invention to provide an apparatus in which the light source and/or the light-receiving means is provide with an auto-focus function of adjusting the focus on the very small area within the specimen contact means.

Yet further, it is an object of the present invention to provide an apparatus including a means for maintaining the distance between the light source and the specimen contact means constant to maintain the very small area constant.

Yet further, it is an object of the present invention to provide an apparatus including a means for introducing the light emitted from the light source into the specimen contact means using an optical fiber.

Yet further, it is an object of the present invention to provide an apparatus in which a lens capable of transmitting ultraviolet rays is used as the light collector means.

Yet further, it is an object of the present invention to provide an apparatus including a means for indicating the measuring position on the specimen contact means.

Yet further, it is an object of the present invention to provide an apparatus in which a magnetic means is used as the means for indicating the measuring position on the specimen contact means.

Yet further, it is an object of the present invention to provide an apparatus including a means for recognizing the measuring position on the specimen contact means.

Yet further, it is an object of the present invention to provide an apparatus including a means for moving a locus position for recognizing the measuring position on the specimen contact means on a locus to a next recognizing locus position with a given distance maintained constant from the original recognizing locus position.

Yet further, it is an object of the present invention to provide an apparatus in which the distance from the current recognizing locus position to the next recognizing locus position is in a range of 10 to 50 µm.

Yet further, it is an object of the present invention to provide an apparatus including a function for recognizing each of signals emitted from microorganisms contained in the specimen contact means as a binary punctual coordinates and counting the number of the punctual coordinates of the signals provided.

Yet further, it is an object of the present invention to provide an apparatus including a function for recognizing adjacent signals of signals provided as the punctual coordinates as one signal.

Yet further, it is an object of the present invention to provide a microorganism counting apparatus in which colored rays emitted from the existing microorganisms can be recognized by receiving them by at least one light-receiving element, and the emitted rays within a given time can be detected to inhibit an error due to the extinction of luminaries, whereby the number of microorganisms can be counted precisely.

Yet further, it is an object of the present invention to provide a microorganism counting apparatus in which the mis-recognition due to colored rays emitted by foreign matters can be prevented by judging the presence of microorganisms when the amounts of colored rays emitted from the existing microorganisms is within preset threshold values, whereby the number of the microorganisms can be counted precisely.

Yet further, it is an object of the present invention to provide a microorganism counting apparatus in which when emitted rays derived from microorganisms are confirmed in the light-receiving element adjoining the periphery of the light-receiving element which has recognized the presence of microorganisms from rays produced from the existing microorganisms, and the rays from the light-receiving element and the adjacent light-receiving elements are judged all together as one group of microorganisms, thereby enhancing the correlation to the prior art process, which is that when colonies are coalesced together with the growth of adjacent microorganisms and recognized as one colony.

It is a further object of the present invention to provide a microorganism counting apparatus in which the number of microorganisms can be measured automatically and promptly by mounting the light source and the light-receiving means.

It is a yet further object of the present Invention to provide a microorganism counting process, wherein an error-influencing factor due to the enzyme activation of microorganisms can be eliminated, whereby the number of microorganisms can be counted precisely.

Further, it is an object of the present invention to provide a microorganism counting process, wherein the integration of microorganisms with a measuring background can be prevented, whereby the common bacteria and particular microorganisms can be measured simultaneously.

Yet further, it is an object of the present invention to provide a microorganism counting process, wherein the wavelength of exciting light and fluorescent ray having a particular wavelength and providing the staining by the exciting light can be controlled by carrying out the staining by fluorescent rays, whereby cells to be detected can be counted precisely.

Yet further, it is an object of the present invention to provide a microorganism counting process, wherein the numbers of living and dead cells can be counted to a one-cell level by using a compound capable of being bonded to nucleic acid in the living and dead cells.

Yet further, it is an object of the present invention to provide a microorganism counting process, wherein the numbers of dead cells can be counted to a one-cell level by using a compound capable of being bonded to nucleic acid in the dead cells.

Yet further, it is an object of the present invention to provide a microorganism counting process, which is capable of counting the number of only living microorganisms by using a compound developing a color by the reaction with a substance derived from microorganisms as a compound for staining living cells.

Yet further, it is an object of the present invention to provide a microorganism counting process, which is capable of judging the presence or absence of living microorganisms at a higher sensitiveness by using a compound developing a color by the reaction with an enzyme protein as a substance derived from microorganisms.

Yet further, it is an object of the present invention to provide a microorganism counting process, which is capable of judging the presence or absence of particular microorganisms at a higher sensitiveness by using a compound developing a color by the reaction with an enzyme protein as a substance derived from particular microorganisms.

Yet further, it is an object of the present invention to provide a microorganism counting process, which is capable of counting the number of particular microorganisms by bringing a compound for staining microorganisms by the reaction with a substance derived from the particular microorganisms into contact with a specimen and detecting the wavelength and the amount of color developed.

### DISCLOSURE OF THE INVENTION

To achieve the above objects, the present invention provides a microorganism detecting kit, comprising a specimen contact means having one or some of the following compounds incorporated therein: a first compound for staining living and dead cells, a second compound for staining dead cells at a wavelength different from the above-described staining, a third compound for staining living cells at a wavelength different from the above-described staining, and at least one or more fourth compounds for staining particular microorganisms at a wavelength different from the above-described staining by the reaction with a substance derived from particular microorganisms. Thus, in the microorganism detecting kit according to the present invention, one type or a plurality of types of staining substrate compounds can be incorporated into a specimen contact portion to achieve the collection and detection of cells simultaneously. Therefore, it is possible to reduce the reagent applying and washing-off steps, and to measure the cell promptly, and when particular microorganisms are detected, it is unnecessary to remove or separate microorganisms other than the particular microorganisms and proteins, which are impurities and hence, the particular microorganisms can be detected promptly.

The present invention provides a microorganism detecting kit, comprising a specimen contact means containing the following compounds incorporated therein: one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms. Thus, in the microorganism detecting kit according to the present invention, the number of the existing cells and the particular microorganisms can be measured simultaneously.

The present invention provides a microorganism detecting kit, comprising a detecting-reagent containing section and a specimen contact means, the detecting-reagent containing section containing one or some of the following compounds incorporated therein: a first compound for staining living and dead cells, a second compound for staining dead cells at a wavelength different from the above-described staining, a third compound for staining living cells at a wavelength different from the above-described staining, and at least one or more fourth compounds for staining particular microorganisms at a wavelength different from the above-described staining by the reaction with a substance derived from particular microorganisms. Thus, in the microorganism detecting kit according to the present invention, the detecting-reagent containing section having a staining substrate is provided in addition to a specimen contact portion and hence, a detecting reagent can be applied to only the specimen contact portion. In addition, depending on the shape of the detecting-reagent containing section, the detecting reagent can be brought promptly into contact with the specimen contact portion, even if the detecting-reagent containing section is not brought into contact with the specimen contact portion. Namely, it is possible to prevent the staining reagent from remaining in the specimen contact portion, and to carry out the operation without touching the specimen contact portion. Further, it is possible to prevent even the incorporation of floating germs in a room.

The present invention provides a microorganism detecting kit, comprising a detecting-reagent containing section and a specimen contact means, the detecting-reagent containing section containing the following compounds incorporated therein: one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms. Thus, in the microorganism detecting kit according to the present invention, it is possible to prevent the staining reagent from remaining in the specimen contact portion, and to carry out the operation without touching the specimen contact portion. Further, it is possible to detect common bacteria and particular microorganisms simultaneously and to prevent the incorporation of microorganisms.

The microorganism detecting kit has a feature that the staining or coloration is fluorescent. Thus, in the microorganism detecting kit according to the present invention, the wavelengths of the fluorescent rays from the compounds are different from one another and hence, the microorganisms can be recognized easily.

The microorganism detecting kit has a feature that the first compound is a compound capable of being bonded to nucleic acid. Thus, in the microorganism detecting kit according to the present invention, living and dead cells can be counted to a one-cell level and at a high accuracy.

The microorganism detecting kit has a feature that the second compound is a compound capable of being bonded to nucleic acid. Thus, in the microorganism detecting kit according to the present invention, dead cells can be counted to a one-cell level and at a high accuracy.

The microorganism detecting kit has a feature that the third compound is a compound capable of developing a color by the reaction with a substance derived from microorganisms. Thus, in the microorganism detecting kit according to the present invention, only living cells can be counted by a difference between amounts of colors developed.

The microorganism detecting kit has a feature that the substance derived from microorganisms is an enzyme protein. Thus, in the microorganism detecting kit according to the present invention, the life or death of the microorganisms can be judged from the reactivity of substance derived from microorganisms by limiting the substance derived from microorganisms to the enzyme protein.

The microorganism detecting kit has a feature that the substance derived from particular microorganisms is an enzyme protein. Thus, in the microorganism detecting kit according to the present invention, the life or death of the particular microorganisms can be judged from the reactivity of substance derived from particular microorganisms by limiting the substance derived from particular microorganisms to the enzyme protein.

The microorganism detecting kit has a feature that at least two or more of the first compound, the second compound, the third compound and the fourth compound are contained in separated states. Thus, in the microorganism detecting kit according to the present invention, even when the staining is carried out at closer wavelengths, the visual judgment can be achieved.

The microorganism detecting kit has a feature that the specimen contact means contains a carrier for supporting at least one or more of the first compound, the second compound, the third compound and the fourth compound. Thus, according to the present invention, it is possible to facilitate the preservation of the microorganism detecting kit by supporting the compound(s) on the carrier, and further, the microorganism detecting kit has -a higher detecting ability by increasing the area of contact with a specimen.

The microorganism detecting kit has a feature that the detecting-reagent containing section contains a carrier for supporting at least one or more of the first compound, the second compound, the third compound and the fourth compound. Thus, according to the present invention, it is possible to facilitate the preservation of the microorganism detecting kit by supporting the oompound(s) on the carrier, and further, the microorganism detecting kit has a higher detecting ability by increasing the area of contact with a specimen. Even when the staining is carried out at closer wavelengths, the visual judgment can be achieved.

The microorganism detecting kit has a feature that the size of at least the specimen contact portion of the specimen contact means is equal to that of the light-receiving means for receiving rays emitted from the specimen contact means. Thus, in the microorganism detecting kit according to the present invention, the scanning is not required.

The microorganism detecting kit has a feature that the specimen contact means has at least one or more of the first compound, the second compound, the third compound and the fourth compound supported on its surface on which such one or more compounds are brought into contact with the specimen. Thus, in the microorganism detecting kit according to the present invention, the amount of the compounds reacting with cells in the specimen can be minimized.

The microorganism detecting kit has a feature that the specimen contact means contains a culture medium for culturing microorganisms. Thus, in the microorganism detecting kit according to the present invention, it is possible to prevent a reduction in activity of microorganisms deposited on the specimen contact means after contact of the compound(s) with the specimen.

The microorganism detecting kit has a feature that the specimen contact means has a self-adherent property on its surface to be brought into contact with the specimen. Thus, in the microorganism detecting kit according to the present invention, the cells deposited to the specimen can be collected reliably.

The microorganism detecting kit has a feature that the specimen contact means has a grip portion. Thus, in the microorganism detecting kit according to the present Invention, even if the specimen has a rugged shape, the self-adhesive layer can be brought into close contact with the surf ace of the specimen. Further, it is possible to prevent an operator from touching the specimen or the specimen contact portion after deposition of the microorganisms to the specimen.

The microorganism detecting kit has a feature that the kit itself has a function for transmitting only light having a particular wavelength. Thus, in the microorganism detecting kit according to the present invention, it is unnecessary to separate rays specially and hence, it is possible to simplify the confirmation of colors developed.

The microorganism detecting kit has a feature that the detecting-reagent containing section has a function for transmitting only light having a particular wavelength. Thus, in the microorganism detecting kit according to the present invention, it is unnecessary to separate rays specially and hence, it is possible to simplify the confirmation of colors developed and to easily carry out the switching placement of a means for separating light having another wavelength.

The microorganism detecting kit has a feature that the specimen is a liquid specimen, and the specimen contact means is a preservation reservoir formed so that the liquid specimen stored in the preservation reservoir is of a given thickness. Thus, in the microorganism detecting kit according to the present invention, even when the specimen is the liquid, the specimen can be introduced into and stored in the preservation reservoir, and the thickness of the specimen can be maintained constant, whereby an error due to a difference in thickness can be reduced.

To achieve the above objects, the present invention provides a microorganism counting apparatus, comprising a specimen contact means in which any one or some of the following compounds are incorporated: a first compound for staining living and dead cells, a second compound for staining dead cells at a wavelength different from the above -described staining, a third compound for staining living cells at a wavelength different from the above-described staining, and at least one or more fourth compounds for staining particular microorganisms at a wavelength different from the above-described staining by the reaction with a substance derived from particular microorganisms; a light source for applying exciting light in a predetermined range of wavelength to a very small given area of the specimen contact means; a light-receiving means for receiving light emitted in a predetermined range of wavelength by virtue of the exciting light; a microorganism judging means adapted to receive the rays emitted by the application of the light from the light source within a preset given time for judging microorganisms, and to determine that microorganisms exist when the amount of light received is in a preset threshold value range; a moving means for moving the very small given area continuously or intermittently; and a means for adding the numbers of microorganisms from signals indicative of the judgment as microorganism from the microorganism judging means. Thus, in the microorganism counting apparatus according to the present invention, it is possible to grasp the living or dead state of the microorganisms contained in the specimen contact means and/or the number or presence or absence of particular germs simultaneously and promptly, and to minimize the area and time of application of the exciting light, thereby inhibiting the extinction of the oompound(s) contained in the specimen contact means by the exciting light. Further, the microorganisms and a foreign matter can be distinguished from each other by providing a threshold value for the intensity of the fluorescent ray, and the counting of the number of microorganisms contained in a given area can be carried out promptly and simultaneously.

In addition, it is possible to detect the microorganisms promptly and easily by incorporating the compound capable of grasping the living or dead state of the microorganisms into the specimen contact means, and it is also possible to avoid the deposition of the detecting-reagent containing section to the specimen by disposing the detecting-reagent containing section and the specimen contact means in a separated relation.

In addition, microorganisms can be recovered efficiently from the specimen by providing a means for transferring microorganisms contained in an object on the surface of the specimen contact moans, and the microorganism counting apparatus has a high sensitiveness.

Even the liquid specimen can be introduced into the preservation reservoir as the specimen contact means, thereby preventing the pollution of the specimen from the outside or the pollution of the outside from the specimen. Further, the area to be irradiated by the exciting light from the light source can be maintained constant by maintaining the liquid specimen at the given thickness, thereby inhibiting the overestimation or the underestimation.

In the microorganism counting apparatus, the staining or coloration is fluorescent, whereby the high-sensitiveness counting can be achieved.

In the microorganism counting apparatus, a compound developing a color by the reaction with a substance produced by the microorganisms (which is the substance derived from the microorganisms) is utilized as the first compound, whereby the activity of the microorganisms can be estimated, and only the living microorganisms can be counted.

In the microorganism counting apparatus, a compound capable of being bonded to nucleic acid is used as the second compound, whereby the dead cells can be detected to a one-cell level at a high sensitiveness.

In the microorganism counting apparatus, a compound capable of being bonded to nucleic acid is used as the third compound, whereby the living cells can be detected to a one-cell level at a high sensitiveness.

In the microorganism counting apparatus, a substance derived from the microorganisms, with which the first compound reacts, is an enzyme protein. In this case, an enzyme reaction is accompanied and hence, microorganisms can be counted at a high sensitiveness.

In the microorganism counting apparatus, a compound developing a color by reaction with a substance derived from particular microorganisms is used as the fourth compound, whereby the particular microorganisms can be detected.

In the microorganism counting apparatus, the substance derived from particular microorganisms is an enzyme protein, which is used as a target for the detection. In this case, an enzyme reaction is accompanied and hence, particular microorganisms can be counted at a high sensitiveness.

In the microorganism counting apparatus, a photoelectric converting element is utilized, thereby enabling the prompt counting, and a collector lens is mounted in front of the photoelectric converting element, thereby enabling the counting at a high sensitiveness.

In the microorganism counting apparatus, photoelectric converting elements are disposed in a linear arrangement, whereby the detection in a given area can be carried out at one time to increase the speed of the counting.

In the microorganism counting apparatus, the specimen contact means is disk-shaped. Thus, it is possible to facilitate the driving of the specimen contact means in a rotating direction and to suppress the distance of detecting movement of both or one of the light source and the light-receiving means to the minimum.

In the microorganism counting apparatus, the specimen contact means is polygonal. Thus, it is possible to facilitate the driving of the specimen contact means in one direction and to count the microorganisms by moving at least one of the light source, the light-receiving means and the specimen contact means.

In the microorganism counting apparatus, the very small area for application of the exciting light emitted from the light source to the specimen is movable in a preset speed range. Thus, it is possible to eliminate the occurrences of the slippage of the color developed, the persistence of vision and the afterglow.

In the microorganism counting apparatus, at least one of the light emitted from the light source and the light emitted from the specimen contact means is collected using a reflecting plate, thereby enabling the application of the light to the specimen contact means at a high efficiency and the detection in the light-receiving means at a high efficiency.

In the microorganism counting apparatus, a light-separating section adapted to transmit only light having a particular wavelength is mounted between the light source and the very small area. Thus, even when a light source adapted to emit light in a wide range of wavelength is used, light having an intended wavelength can be applied.

In the microorganism counting apparatus, a light-separating section adapted to transmit only light of a particular wavelength is mounted between the very small area and the light-receiving means. Thus, even when the staining is carried out by light having a wavelength in a wide range, fluorescent rays having an intended wavelength can be detected.

The microorganism counting apparatus includes a function of automatically adjusting the focus on the very small detection area in the specimen contact means. Thus, it is possible to detect the microorganisms existing on the regularly constant area at all times and to enhance the operator's operability.

In the microorganism counting apparatus, an optical fiber is utilized to introduce the exciting light from the light source. Thus, it is possible to place the light source being as a heat source at another location. Further, even when the specimen contact means is of a complicated shape, a regularly constant amount of light can be introduced into the specimen contact means.

In the microorganism counting apparatus, an ultraviolet ray-transmittable lens is used as a collector means for light emitted from the light source. Thus, it is possible to introduce exciting light having a wavelength in an ultraviolet range into the specimen contact means without reduction in amount of light.

In the microorganism counting apparatus, a means for indicating the measuring position on the specimen contact means is provided. Thus, it is possible to recognize a detecting position.

In addition, it is possible to recognize the detecting position without being influenced by the size, the shape and the like of the specimen contact means by provision of the means for indicating the measuring position on the specimen contact means.

Further, it is possible to recognize the detecting position in accordance with the type of the specimen contact means by provision of the means for indicating the measuring position on the specimen contact means.

In the microorganism counting apparatus, the means for indicating the measuring position on the specimen contact means is a magnetic means. Thus, it is possible to recognize the detecting position on the specimen contact means simply and precisely.

The microorganism counting apparatus includes a means for recognizing the measuring position on the specimen contact means. Thus, it is possible to regulate the detecting position on the specimen contact means.

The microorganism counting apparatus includes a means for moving the measuring position on the specimen contact means from a current recognizing locus position to a next recognizing locus position with the distance kept constant. Therefore, it is unnecessary to detect the entire specimen and hence, it is possible to increase the counting speed.

In the microorganism counting apparatus, the distance from the locus position in which the measuring position on the specimen contact means has been recognized to the next recognizing locus position is in a range of 10 to 50 µm. Thus, it is possible to carry out the counting in a high correlation to the culture process.

In the microorganism counting apparatus, the signal provided from microorganisms contained in the specimen contact means is recognized as a binary punctual coordinates. Thus, it is possible to carry out the counting easily and promptly.

In the microorganism counting apparatus, adjacent signals of signals provided as the punctual coordinates are recognized as one signal. Thus, it is possible to carry out the counting in a high correlation to the culture process.

Further, the present invention provided a microorganism counting apparatus for counting microorganisms utilizing colored and emitted rays, comprising a light-receiving means adapted to receive the emitted rays by at least one light-receiving element, the rays being detected within a given time to judge the presence of microorganisms from the amounts of rays detected by the light-receiving element to count the microorganisms in an adding manner. In the microorganism counting apparatus according to the present invention, the emitted rays can be recognized by the reception thereof by the light-receiving element and detected within the given time. Thus, it is possible to inhibit an error due to the extinction of luminaries and to count the number of the microorganisms precisely.

The microorganism counting apparatus for counting microorganisms utilizing colored and emitted rays has a feature that when the amounts of rays when being recognized by the light-receiving means after the coloration and the emission of rays is fallen within preset threshold values, such rays can be determined as being emitted from microorganisms. Thus, in the microorganism counting apparatus according to the present invention, it is possible to count the number of microorganisms precisely without recognition of over-emission or under-emission of rays from foreign matters.

The microorganism counting apparatus for counting microorganisms utilizing colored and emitted rays has a feature that when rays emitted from microorganisms and received by a light-receiving element and rays entering a light-receiving element adjoining such light-receiving element have been recognized as microorganisms, these microorganisms are judged all together as one group of microorganisms, thereby maintaining the correlation to the prior art process, which is that when microorganisms are cultured, colonies are coalesced together with the growth of adjacent microorganisms and recognized as one colony.

The microorganism counting apparatus includes a light source and a light-receiving means, whereby cells are counted based on a difference between wavelengths of rays from the specimen and the amounts of fluorescent colors developed. Thus, according to the present invention, the microorganisms can be counted more promptly and precisely.

To achieve the above objects, the present invention provides a microorganism counting process comprising the steps of bringing a first compound for staining living and dead cells and a second compound for staining dead cells at a wavelength different from the above-described staining into contact with a specimen, and detecting both of living cells and dead cells simultaneously from a difference between wavelengths and a difference between amounts of colors developed. Thus, according to the present invention, the measurement can be achieved at a high sensitiveness even in environments where a large number of types of microorganisms exist, and the estimation can be achieved even in actual environments. Therefore, the measurement and the estimation are not influenced by substances within the microorganisms and substances discharged out of microorganism cells and hence, the numbers of living and dead cells can be grasped precisely, and all the cells can be detected, because the non-specific bonding is utilized in the present technique. In addition, the life and death of the cells can be discriminated from each other. Further, the compound for staining the living and dead cells and the compound for staining the dead cells are high in stability and easy to preserve and can be used even in environments such as at a lower temperature.

Further, the present invention provides a microorganism counting process comprising the step of bringing one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-desoribed staining by the reaction with a substance derived from particular microorganisms, into contact with a specimen, thereby detecting both or either one of the living cells and the dead cells, and the particular microorganisms simultaneously from a difference between wavelengths and amounts of colors developed. Thus, in the microorganism counting process according to the present invention, the particular microorganisms can be measured at a high sensitiveness from the multiple of types of cells and microorganisms, and the estimation can be achieved even in actual environments. The substance derived from the particular microorganisms is measured, and hence, the measurement and estimation are not influenced by substances in other cells and microorganisms and substances discharged out of the microorganism cells. Therefore, the presence of the microorganisms can be confirmed and further, the microorganisms can be measured promptly without need for separating and washing means for pretreatments.

The microorganism counting process has a feature that the staining or coloration is fluorescent. Thus, according to the present invention, only the microorganisms stained by fluorescent rays can be counted at a high sensitiveness.

Further, the microorganism counting process has a feature that the first compound is a compound capable of being bonded to nucleic acid. Thus, according to the present invention, it is possible to enable the counting of the living and dead cells to a one-cell level and at a high sensitiveness.

Further, the microorganism counting process has a feature that the second compound is a compound capable of being bonded to nucleic acid. Thus, according to the present invention, it is possible to enable the counting of the dead cells to a one-cell level and at a high sensitiveness.

Further, the microorganism counting process has a feature that the third compound is a compound for staining microorganisms by the reaction with a substance derived from microorganisms in the specimen. Additionally, the microorganism counting process has a feature that the substance derived from microorganisms is an enzyme protein. Thus, according to these inventions, it is possible to count the number of only living microorganisms, and further it is possible to judge the presence or absence of living microorganisms at a higher sensitiveness by using the enzyme protein as the substance derived from microorganisms.

Yet further, the microorganism counting process has a feature that the substance derived from the particular microorganisms is an enzyme protein. Thus, according to the present invention, it is possible to recognize the presence or absence of the particular microorganisms by detecting the substance derived from the particular microorganisms appearing within or outside the cells of the particular microorganisms. Further, it is possible to recognize the presence or absence of the particular microorganisms at a higher sensitiveness by limiting the substance derived from the particular microorganisms to the enzyme protein.

Yet further, the present invention provides a microorganism counting process comprising the step of bringing acompoundfor stainingparticular microorganisms by the reaction with a substance derived from particular microorganisms into contact with a specimen to detect the particular microorganisms from a wavelength and an amount of color developed. Thus, according to the present invention, it is possible to reliably recognize the particular microorganisms by specifically bonding the compound to the particular microorganisms and further to precisely grasp the number of particular microorganisms from an amount of fluorescent color different from the color of the reagent.

### BRIEF DESCRIPTION OF THE DRAWINGS

Fig.1 is an illustration of the arrangement of a microorganism detecting kit according to Example 1 of the present invention.
Fig. 2 is an illustration of the arrangement of a detecting kit according to Example 2 of the present invention, which includes a reagent-containing vessel and a specimen contact vessel.
Fig.3 is an illustration of the arrangement of a detecting kit including reagent-containing sheets.
Fig.4 is an illustration of the arrangement of a detecting kit with carriers retained on each of reagent-containing sheets according to Example 3 of the present invention.
Fig. 5 is an illustration of the arrangement of a detecting kit with reagent-supporting carriers retained in a separated manner on each of reagent-containing sheets according to Example 4 of the present invention.
Fig.6 is an illustration of the arrangement of a detecting kit including a light-separating film according to Example 5 of the present invention.
Fig.7 is an illustration of the arrangement of a liquid specimen detecting kit according to Example 6 of the present invention.
Fig.8 is a curve of attenuation of fluorescent light according to Example 7 of the present invention.
Fig. 9 is a diagram indicating threshold values with respect to the intensity of fluorescent light according to Example 8 of the present invention.
Fig.10 is an illustration of the arrangement of a bacterium- recognizing means according to Example 9 of the present invention.
Fig.11 is a diagram of a locus A of exciting light on a specimen according to Example 10 of the present invention.
Fig.12 is a diagram of a locus B of exciting light on the specimen according to Example 10 of the present invention.
Fig.13 is a diagram showing a waveform model according to Example 10 of the present invention.
Fig.14 is a diagram showing a waveform model according to Example 10 of the present invention, when there are microorganisms existing adjacent one another.
Fig.15 is a fluorescent ray extinction model according to Example 10 of the present invention.
Fig.16 is a diagram showing a microorganism counting apparatus according to Example 10 of the present invention.
Fig. 17 is a diagram showing a binary model according to Example 10 of the present invention.
Fig.18 is a diagram showing a microorganism counting apparatus according to Example 11 of the present invention.
Fig.19 is a diagram showing a specimen contact means according to Example 12 of the present invention.
Fig.20 is a diagram for explaining a process for counting living and dead cells and dead cells according to Example 13 of the present invention.
Fig.21 is a block diagram of a microorganism counting apparatus according to Example 14 of the present invention.
Fig.22 is a graph showing a calibration curve for living and dead cells and electric signal output in Examples 13 and 14.
Fig.23 is a graph showing a calibration curve for dead cells and electric signal output in Examples 13 and 14.
Fig. 24 is a diagram for explaining a process for counting living and dead cells, dead cells, living cells and Coliform bacteria according to Example 15 of the present invention.
Fig.25 is a graph showing a calibration curve for living cells having a high activity and electric signal output according to Examples 15 and 16 of the present invention.
Fig. 26 is a graph showing a calibration curve for Coliform bacteria and electric signal output according to Examples 15 and 16 of the present invention.
Fig.27 is an illustration of the arrangement of a prior art detecting kit.
Fig.28 is a view of the arrangement of a prior art microorganism measuring apparatus.

### BEST MODE FOR CARRYING OUT THE INVENTION

A microorganism detecting kit according to the present invention includes a specimen contact means having one or some of the following compounds incorporated therein: a first compound for staining living and dead cells , a second compound for staining dead cells at a wavelength different from the above-described staining, a third compound for staining living cells at a wavelength different from the above-described staining, and at least one or more fourth compounds for staining particular microorganisms at a wavelength different from the above-described staining by the reaction with a substance derived from particular microorganisms. The specimen contact means, for example, is comprised of a self-adhesive layer retaining a detecting reagent thereon and adapted to collect microorganisms from a specimen, a support for supporting the self-adhesive layer, and a grip which is in contact with the support to adjoin the latter. The microorganism detecting kit has such an effect that only living and dead cells can be stained by bonding of the compound for staining the living and dead cells to nucleic acid in the living and dead cells, and the presence or absence of the living and dead cells can be detected, or the numbers of the living and dead cells can be counted. The self-adhesive layer, for example, is one formed by mixing a solution resulting from mixing of peptone, sodium chloride or the like with an agar suspension, with 4' ,6-diamidino-2-phenylindole dihydrochloride used as a compound for staining living and dead cells, and placing the solution containing the staining compound into a vessel after sterilization to solidify the solution. If the specimen is a wall surface, living and dead cells deposited to the wall surface are deposited to a surface of a culture medium containing the compound by bringing the surface of the self-adhesive layer into contact with the wall surface using the grip.

Another microorganism detecting kit according to the present invention includes a specimen contact means containing the following compounds incorporated therein: one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms. For example, when microorganisms are to be detected for food sanitation, it is a conventional practice that the number of common bacteria is counted, and the presence or absence of Coliform bacteria is confirmed. In such a case, the detection kit according to the present invention has such an effect that it is possible to simply carry out the counting of the common bacteria and the confirmation of the presence or absence of Coliform bacteria simultaneously and promptly, by counting microorganisms, i.e., living cells, which can be cultured on a culture medium, and by using a culture medium, in which a compound which can react with a substance derived from particular microorganisms which is Coliform bacteria, is mixed.

A further microorganism detecting kit according to the present invention includes a detecting-reagent containing section and a specimen contact means, the detecting-reagent containing section containing one or some of the following compounds incorporated therein: a first compound for staining living and dead cells, a second compound for staining dead cells at a wavelength different from the above-described staining, a third compound for staining living cells at a wavelength different from the above-described staining, and at least one or more fourth compounds for staining particular microorganisms at a wavelength different from the above-described staining by the reaction with a substance derived from particular microorganisms. Thus, living and dead cells deposited can be stained, and the presence of the living and dead cells can be judged, or the numbers of the living and dead cells can be counted. Further, it is possible to prevent the detecting reagent from remaining on the surface of the specimen by carrying out this operation. The specimen contact means, for example, is comprised of a self-adhesive layer adapted to collect microorganisms from the specimen, a support for supporting the self-adhesive layer, and a grip which is in contact with the support to adjoin the latter. The detecting-reagent containing section is comprised of a film or a faoe-shaped layer containing the detecting reagent, and a support. The specimen contact means and the detecting-reagent containing section are connected to each other, for example, by a hinge, so that the self-adhesive layer of the specimen contact means and the film or the face-shaped layer of the detecting-reagent containing section are inner surfaces and in contact with each other. Alternatively, the specimen contact means and the detecting-reagent containing section may be formed in separated states. For example, the specimen contact portion is of a structure in which a generally commercially available normal agar culture medium (made by Nissui Seiyaku, Co.) is used as the self-adhesive layer and laminated by the support for supporting the self-adhesive layer, and a grip is attached to adjoin the support. The detecting-reagent containing section is comprised of the film having 4',6-diamidino-2-phenylindole dihydrochloride retained thereon as the compound for staining the living and dead cells, and the support, so that a specimen is brought into contact with the surface of the self-adhesive layer of the specimen contact means using the grip, whereby cells deposited to the specimen are collected onto the surface of the self-adhesive layer. After the collection, the film retaining the compound in the detecting-reagent containing section is affixed to the cell-deposited surface of the self-adhesive layer, and the detecting reagent retained in the detecting-reagent containing section is moved onto the self-adhesive layer.

A further microorganism detecting kit according to the present invention includes a detecting-reagent containing section and a specimen contact means, the detecting-reagent containing section containing the following compounds incorporated therein: one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms. Thus, living and dead cells deposited can be stained, and the presence of the living and dead cells can be judged, or the numbers of the living and dead cells can be counted. Further, the presence or absence of Coliform bacteria can be confirmed simultaneously. Namely, the number of common bacteria and the presence or absence of Coliform bacteria can be confirmed easily and promptly. Further, the microorganism detecting kit has an effect that it is possible to prevent the detecting reagent from remaining on the surface of the specimen by this operation. When the number of common bacteria is to be counted and the presence or absence of Coliform bacteria is to be confirmed, the specimen contact means is formed using a culture medium comprising a commercially available normal agar culture medium, and the detecting-reagent containing section is formed using a film retaining 4' , 6-diamidino-2-phenylindole dihydrochloride as the compound for staining the living and dead cells and a compound reactive with a substance derived from Coliform bacteria. Thus, the specimen is brought into contact with the surface of the normal agar culture medium, whereby cells deposited to the specimen are collected. After the collection, the film retaining the compounds thereon is affixed to the cell-deposited surface of the normal agar culture medium, and the detecting reagent retained is moved onto the surface of the normal agar culture medium.

In the microorganism detecting kit, the staining or coloration is fluorescent. In this case, the color of the detecting reagent covering the surface of the culture medium and the fluorescent color developed are different from each other. Therefore, the microorganism detecting kit has an effect that it is possible to prevent the interference of the staining wavelength, to easily recognize the microorganisms even visually, and further to count the microorganisms precisely.

In the microorganism detecting kit, the first compound is a compound capable of being bonded to nucleic acid. In this case, the dyeing of the nucleic acid exiting within cells is utilized as an indication and hence, after the dyeing, the cells can be detected to a one-cell level. Thus, the microorganism detecting kit has an effect that the numbers of living and dead cells can be counted at a further high sensitiveness.

In the microorganism detecting kit, the second compound is a compound capable of being bonded to nucleic acid. In this case, the dyeing of the nucleic acid exiting within cells is utilized as an indication and hence, after the dyeing, the cells can be detected to a one-cell level. Thus, the microorganism detecting kit has an effect that the numbers of dead cells can be counted at a further high sensitiveness.

In the microorganism detecting kit, the third compound is a compound suitable to stain microorganisms by reacting with a substance derived from microorganisms. In this case, the compound reacts with a metabolite derived from living cells, thereby providing an effect that the living or dead of the cells can be judged by the reactivity.

In the microorganism detecting kit, the substance derived from the microorganisms is an enzyme protein. In this case, the microorganism detecting kit has an effect that the living or dead of the living cells can be judged by the reactivity.

In the microorganism detecting kit, the substance derived from the particular microorganisms is an enzyme protein. In this case, the microorganism detecting kit has an effect that the living or dead of the particular microorganisms can be judged by the reactivity.

In the microorganism detecting kit, at least two or more of the first compound, second compound, third compound and fourth compound are contained in separated states. In this case, the microorganism detecting kit has an effect that the compounds staining the microorganisms at adjacent wavelengths by the reaction can be confirmed visually.

The microorganism detecting kit includes the specimen contact means which contains a carrier for supporting at least one or more of the first compound, second compound, third compound and fourth compound. In this case, the microorganism detecting kit has an effect that it is possible to facilitate the preservation of the compounds by supporting them on the carrier, and to enhance the detecting sensitiveness by increasing the area of contact with the specimen.

The microorganism detecting kit includes the detecting-reagent containing section which contains a carrier for supporting at least one or more of the first compound, second compound, third compound and fourth compound. In this case, the microorganism detecting kit has an effect that it is possible to facilitate the preservation of the compounds by supporting them on the carrier, to enhance the detecting sensitiveness by increasing the area of contact with the specimen, and to visually confirm the compounds staining the microorganisms at adjacent wavelengths by the reaction by separating the carriers supporting each compounds.

In the microorganism detecting kit, the size of the specimen contact portion is equal to that of a light-receiving means. In this case, the microorganism detecting kit has an effect that a scanning course can be omitted by the fact that the size of the specimen contact portion is equal to that of the light-receiving means , and the microorganisms can be detected more promptly.

In the microorganism detecting kit, the specimen contact means having at least one or more of the first compound, second compound, third compound and fourth compound contained on its surface to be brought into contact with the specimen. Thus, the microorganism detecting kit has an effect that it is possible to enhance the reactivity with microorganisms contained in the specimen by supporting compound(s) only on the specimen contact portion.

In the microorganism detecting kit, the specimen contact means contains a culture medium for culturing microorganisms. Thus, the microorganism detecting kit has an effect that it is possible to maintain the activity of cells, or increase the reduced activity of the cells to enhance the reactivity.

In the microorganism detecting kit, the specimen contact means has a self-adherent property on its surface to be brought into contact with the specimen. Thus, the microorganism detecting kit has an effect that cells can be collected reliably by the fact that the specimen contact portion has the self-adherent property.

In the microorganism detecting kit, the specimen contact means is provided with a grip portion. Thus, the microorganism detecting kit has an effect that microorganisms can be collected irrespective of the shape of the specimen and further, it is possible to prevent an operator from touching the specimen or the specimen contact portion after deposition of microorganisms to the specimen.

The microorganism detecting kit itself has a function to transmit only light having a particular wavelength. Thus, the microorganism detecting kit has an effect that a special light-separating means is not required and hence, it is possible to simplify the confirmation of a color developed.

The microorganism detecting kit Includes the detecting-reagent containing section having a function to transmit only light having a particular wavelength. Thus, since the detecting-reagent containing section has the light-separating function, the microorganism detecting kit has an effect that a special light-separating means is not required and hence, it is possible to simplify the confirmation of a color developed, and it is possible to easily conduct the switching placement of a means for separating light having another wavelength.

In the microorganism detecting kit, the specimen is a liquid specimen, and the specimen contact means is a preservation reservoir for preserving the specimen, so that the liquid specimen stored in the preservation reservoir is of a given thickness. The microorganism detecting kit has a function to be able to preserve even the liquid specimen under constant conditions, whereby the detection can be simplified. Further, the microorganism detecting kit has an effect that it is possible to prevent the mis-recognition due to a variation in thickness by setting the thickness of the liquid specimen at the given value and hence to carry out the precise detection.

A microorganism counting apparatus according to the present invention is intended to count microorganisms contained in a specimen, and includes a specimen contact means having such a shape that it can be easily brought into contact with a wall surface or a cookware such as a shape like a food stamp or a film shape, or having a structure provided with a grip. Any one or some of the following compounds are previously incorporated into the specimen contact means: a first compound for staining living and dead cells, a second compound for staining dead cells at a wavelength different from the above-described staining, a third compound for staining living cells at a wavelength different from the above-described staining, and at least one or more of fourth compounds for staining particular microorganisms at a wavelength different from the above-described staining by the reaction with a substance derived from particular microorganisms. The compound(s) incorporated into the specimen contact means can be changed depending on the purpose, whereby microorganisms to be detected can be changed. For example, when microorganisms to be detected are only living germs, only the third compound is incorporated, and when the sterilizing effect is to be verified, the second and third compounds are incorporated. Thus, the compounds can be selected as desired, depending on the purpose and the microorganisms to be detected. The specimen contact means, if it is of a culturing type usually used, is of a size on the order of 40 mm in diameter. However, if the size of germs contained in the specimen contact means are as very small as 1 to 5 µm, and a CCD camera conventionally used is employed to conduct the picture processing, the number of required pixels is enormous, and it is difficult to conduct the direct recognition. For this reason, such germs could be never detected without use of a magnifying means having a magnification of about 1000. Therefore, the present inventors have aimed at the size of germs contained in a surface of the specimen contact means in contact with a specimen, and have found that exciting light is applied in a predetermined range of wavelength to the area of 1 to 5 µm square (which is desired to be as large as possible, if it can be measured as a luminance of a light-receiving means, but which is desired to be equal to or smaller than the same size of 30 µm square as a photodiode), and the amount of rays emitted by virtue of the exciting light is measured, whereby the number of microorganisms is counted from a value resulting from the addition of the rays. In this case, the intensity of fluorescent ray emitted by application of the exciting light is changed with the time. Therefore, it is necessary to set a time within a given range of amount of rays emitted, and to judge the presence or absence of microorganisms from the amount of rays at that time. More specifically, impurities other than microorganisms emit no fluorescent ray, but emit scattered light of exciting light or the like, which provides an influence upon reception of light. For this reason, when the amount of rays received has reached equal to or larger than a given value, it is determined that there are microorganisms. Depending on the type of impurities, it can be assumed that the amount of fluorescent rays emitted from objects may be equal to or more than the amount of rays emitted from microorganisms. For this reason, if an amount of rays is in a preset range, it is determined that there are microorganisms, and basically, if an amount of rays in the range is received, it is determined that there is one microorganism. A very small area, to which the exciting light is applied, is moved continuously or intermittently, and for example, as compared with data in a conventional agar culture medium diffusion process, the entire area having a diameter of 40 mm is scanned, and the numbers of microorganisms in such areas are added, whereby the total number of microorganisms can be counted. Therefore, it is possible to prevent the underestimation due to the extinction of the compound(s) by the exciting light and to prevent the overestimation due to rays emitted from foreign matters. Thus, it is possible to confirm the life or death of microorganisms in a short time and to count the numbers of living and dead microorganisms in a short time without need for special technique and equipment, as compared with the conventional culture process and antibody process.

It is of course that a reagent as harmless as possible is selected as each of the first, second, third and fourth compounds. However, the microorganism counting apparatus is used mainly in food factories in many oases, and when what is to be detected is a cookware, it is not preferred for sanitation that a specimen contact means containing a reagent, even if it is harmless, is brought into contact with the cookware. In some oases, it is preferable depending on the characteristics of a reagent that the reagent is allowed to react with a specimen immediately before application of exciting light, and that each of the first, second, third and fourth compounds is carried on a film independently of the specimen contact means, or stored in a vessel and added to the specimen contact means immediately before application of exciting light. Based on this viewpoint, 4' ,6- diamidino-2-phenylindole, which is one of reagents capable of being permeated non-specifically into living and dead cells through their membrane surfaces and bonded specifically to nucleic acid existing in cells, is retained in the deteoting-reagent containing section, so that it is permeated from the detecting-reagent containing section into microorganisms collected on the specimen contact means. The life or death of microorganisms collected can be judged at the same time by doubly dyeing the microorganisms using propidium iodide, which is one of reagents capable of being permeated non-specifically into dead cells through their membrane surfaces and bonded specifically to nucleic acid existing in cells, and applying exciting light having a specific wavelength to the microorganisms to stain or color the microorganisms. Alternatively, living microorganisms can be detected directly by applying exciting light having a specific wavelength to a compound capable of being permeated into cells and decomposed by esterase existing in living microorganism cells to stain the microorganisms, as is 6-carboxyfluorescein diacetate, thereby staining or coloring the living microorganisms. Additionally, it can be confirmed promptly whether microorganisms to be detected exist in microorganisms sampled from a specimen, by using a detecting-reagent containing section containing a compound reactive with a substance derived from particular microorganisms and a microorganism contact means.

Microorganisms existing in a specimen are deposited efficiently to the specimen contact means by utilizing a transfer means. The transfer means includes those having a viscosity and liable to be adhered in a lattice configuration. It is possible to prevent the underestimation of microorganisms contained in a specimen by detecting the microorganisms using a specimen contact means having such a transfer means.

Even when the specimen is liquid, the detection of microorganisms is made possible by utilizing a preservation reservoir for preserving the specimen. Examples of the preservation reservoirs are those defined by a thin layer formed at a site where a specimen is added, and the specimen is introduced to the preservation reservoir and spread, whereby microorganisms contained in the specimen are uniformized and detected. Alternatively, a fine groove may be defined previously in the preservation reservoir, and a liquid specimen may be poured into the fine groove, whereby the liquid specimen can be spread in the preservation reservoir. Further, by using a specimen contact means formed so that a liquid specimen stored in the preservation reservoir is of a given thickness, it is possible to maintain the distance from the light source or the light-receiving means to the specimen constant and to carry out the estimation with less error.

There is also an effect that microorganisms can be detected at a high sensitiveness and a very small specimen can be also detected, by the fact that the staining or coloration is fluorescent.

In addition, the compound for staining or coloring the living cells is a compound for staining or coloring the living cells by reacting with a substance derived from microorganisms, and an amount of such substance produced is detected, whereby the activity of microorganisms can be estimated. In this case, it is possible to grasp the number of the living cells by counting the stained microorganisms.

The compound for staining the living and dead cells or the dead cells is a compound capable of being bonded to nucleic acid. Thus, the nucleic acid retained in the cells can be detected and hence, the cells can be detected to acne-cell level, and the number of existing microorganisms can be counted.

In addition, by targeting the substance derived from particular microorganisms, the particular microorganisms can be detected. Further, by targeting an enzyme protein, the detection of the particular microorganisms can be achieved at a high sensitiveness, and the activity of the particular microorganisms can be also grasped.

The light-receiving means is at least one photoelectric converting element, and a collector lens for collecting rays emitted by virtue of the exciting light is mounted in front of the photoelectric converting element. This provides the following effect: Fluorescent rays generated by the exciting light can be introduced in an increased range of the fluorescent rays into the photoelectric converting element and hence, a high detection sensitivity can be maintained.

A plurality of photoelectric converting elements included in the light-receiving means are disposed in a linear arrangement. Thus, the movement of the photoelectric converting elements in a direction of an X-axis (a direction of arrangement of the photoelectric converting elements) is not required, and the detection of microorganisms in a given area can be carried out by moving the photoelectric converting elements only in a direction of a Y-axis (in a moving direction), and hence, the counting of microorganisms can be carried out promptly.

The specimen contact means is of a disk shape and is movable. Thus, the detection of microorganisms can be achieved by rotating the specimen or the light source. For example, a photo-disk reading device may be used as a detecting means. The movement of the light source can be carried out at only the radius of the specimen contact means by placing and rotating the specimen contact means. Thus, the detection can be automated and increased in speed.

The specimen contact means may be of a polygonal shape and is movable. Therefore, microorganisms contained on the specimen contact means can be detected easily. For example, a scanner may be used as a detecting means. Thus, the detection can be automated and increased in speed by placing and moving the specimen contact means.

A drive means for moving either one or some of the light source, the light-receiving means and the specimen contact means is mounted as a means for moving a very small area and is movable in a preset range of speed. Thus, it is possible to prevent the occurrence of the slippage of the color developed, the occurrence of the persistence of vision or the occurrence of the afterglow at the time of moving a very small area, and to prevent the overestimation or the underestimation.

A reflecting plate is mounted as a means for collecting light emitted from the light source and/or a means for collecting light to the light-receiving means. This provides an effect that it is possible to prevent a loss of extra light and to enhance the sensitiveness by collecting the light.

A light-separating section for transmitting light having a particular wavelength is mounted between the light source and the very small area. Thus, even when the wavelength of light emitted from the exciting light source is wide, the exciting light having the particular wavelength can be extracted. Further, by mounting the light-separating section for transmitting light having a particular wavelength between the very small area and the light-receiving means, it is possible to extract fluorescent rays having a particular wavelengths among the fluorescent rays having the various wavelengths, as does the exciting light, and the counting can be carried out at a high accuracy.

An auto-focusing means for adjusting the focus on the very small area within the specimen contact means is mounted on at least one of the light source and the light-receiving means. Thus, the stable counting can be achieved without dependence on the shape of the surface of the specimen contact means. Further, by the provision of the auto-focusing means, the area irradiated by the exciting light can be maintained constant, and the load applied to microorganisms collected by the specimen contact means and stained or colored can be minimized, thereby avoiding the underestimation.

A means is provided for introducing light emitted from the light source into the specimen contact means using an optical fiber. Thus, even if the specimen contact means is of a complicated shape, stable exciting light can be provided. Further, the light source can be mounted at another location by using the optical fiber. Thus, even when an exothermic light source is used, the load applied to the specimen contact means and microorganisms collected by the specimen contact means due to a generated heat can be minimized.

By using a lens adapted to transmit ultraviolet rays, an ultraviolet ray-generating means can be used as the light source, and hence, even exciting light in an ultraviolet range can be provided stably to the specimen contact means.

The apparatus or the specimen contact means may be provided with a means for indicating a measuring position, and the light-receiving means is provided with a means for recognizing the measuring position. Thus, it is possible to grasp a position in which at least one of the light source and the light-receiving means exists, to prevent the double detection of such position and the omission of detection and to avoid the overestimation or the underestimation. Further, the means for indicating the measuring position on the specimen contact means is magnetic, thereby providing an effect that the position can be recognized simply and precisely, and the overestimation or the underestimation can be avoided.

When the position on the locus for recognizing the measuring position on the specimen contact means is to be moved, it is moved to a next recognizing locus position with the distance kept constant from the original recognizing locus position. This makes it possible to prevent the omission of detection and to detect the entire specimen contact means. Further, the distance from the original recognizing locus position to the next recognizing locus position is in a range of 10 to 50 µm, thereby providing an effect that it is possible to enhance the correlation to the culture process, which is one of the conventional processes. This distance is a minimum distance capable of avoiding a range of recognizing colonies as one colony due to the duplication of the colonies, as is the case with the culture process.

Microorganisms contained in the specimen contact means can be counted easily and promptly by recognizing each of signals provided from the microorganisms contained in the specimen contact means as a binary punctual coordinates, and counting the number of the punctual coordinates of the provided signals. Further, a luminance is determined from the binary punctual coordinates, whereby the activity of the microorganisms can be measured. Additionally, the correlation to the culture process can be enhanced by recognizing the adjacent signals as one signal. Yet further, the shape of deposits deposited to the specimen contact means can be grasped from the adjacent signals, and objects of shapes other than microorganisms can be eliminated from the counting, leading to an enhancement in accuracy.

In a microorganism counting apparatus for counting microorganism utilizing the staining emission of light, received light is received by a light-receiving element, and the number of microorganisms is determined from an amount of light within a given time. Luminaries can be recognized by provision of a light-receiving means for receiving the colored and emitted rays. Further, by limiting the rays to an amount of light detected within the given time by the light-receiving element, it is possible to prevent the mis-recognition caused upon the extinction of microorganisms stained to emit the light and to recognize the precise colored and emitted rays, and to recognize the number of microorganisms precisely by adding and counting such rays.

In the microorganism counting apparatus for counting microorganisms utilizing colored and emitted rays, preset threshold values are provided for the intensity of the colored and emitted rays received by the light-reoeiving means, and when the intensity of the colored and emitted rays is fallen within the range of the threshold values, it is determined that there are microorganisms. Thus, too large or too small coloration due to foreign matters can be distinguished from the coloration of microorganisms, and the number of microorganisms can be recognized precisely by adding and counting the emitted rays within the threshold values.

In the microorganism counting apparatus for counting microorganisms utilizing colored and emitted rays, when rays emitted by microorganisms and recognized by a light-receiving element and rays entering a light-receiving element adjoining such light-receiving element are recognized as the presence of microorganisms, the microorganisms are judged all together as one group of microorganisms. Thus, it is possible to maintain the correlation to a case where when adjacent microorganisms form colonies with the growth thereof, the adjacent colonies are coalesced together and recognized as one colony in the plate counting process, which is one of the prior art processes.

The apparatus includes a light source and a light-receiving means, so that cells are counted by a difference between wavelengths of rays emitted by a specimen and amounts of fluorescent colors developed. This apparatus has an effect that cells emitting fluorescent rays can be counted simply, and the estimating time can be shortened.

A microorganism counting process according to the present invention comprises the steps of bringing a first compound for staining living and dead cells and a second compound for staining dead cells at a wavelength different from the above-described staining into contact with a specimen, and detecting the living cells and the dead cells simultaneously from a difference between the wavelengths and a difference between amounts of colors developed. Thus, the cells can be measured at a high sensitiveness even in environments where there are many types of microorganisms, and the estimation can be achieved even in actual service environments, without dependence on the enzyme activity indicated by the microorganisms.

Even when an enzyme is produced outside cells of microorganisms by the microorganisms, the microorganisms can be detected, while preventing the integration of the microorganisms with the background, because the compounds do not react the enzyme.

In addition, unlike the detection using an antibody, the living and death of cells can be distinguished from each other at a high sensitiveness, because the compounds are bonded specifically to many types of cells. For example, by counting living and dead cells and dead cells simultaneously by bringing a reagent comprising of a mixture of 4',6-diamidino-2-phenylindole dihydrochloride capable of being bonded to nucleic acid contained within living and dead cells with a propidium iodide capable of being bonded to nucleic acid contained within dead cells into contact with a specimen, and the number of the living cells can be measured from a difference between the number of the living and dead cells and the number of the dead cells. Further, the reagent reacts with individual cells quickly to achieve the staining or coloration and hence, the presence of the living and dead cells can be confirmed within a short time, as compared with the conventional culture process and the conventional antibody process.

The microorganism counting process comprises the steps of bringing one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms, into contact with a specimen, and detecting both or one of the living cells and the dead cells and the particular microorganisms simultaneously from a difference between the wavelengths and amounts of colors developed. The living and dead cells, the dead cells, the living cells and the particular microorganisms can be detected simultaneously, whereby the counting of polluting microorganisms in medical and food sanitation fields and the monitoring of microorganisms in environments can be carried out promptly. For example, the number of common bacteria can be counted in food fields by the counting of the living and dead cells, the counting of the dead cells and the counting of the living cells and further, the states of foods after being sterilized can be grasped by measuring the dead cells. In addition, because Coliform bacteria discharge a specific enzyme protein called β-galactosidase, the presence of Coliform bacteria can be ascertained by using 4-methylumbelliferyl-β-D-galaotoside, which reacts with the enzyme protein, and hence, the microorganism pollution can be examined in medical and food sanitation fields.

The staining or coloration is fluorescent, thereby providing an effect that the interference of the wavelength of colored rays can be prevented, and the colored rays can be visually recognized simply. Further, the precise number of cells can be counted.

The first compound is one capable of being bonded to nucleic acid. Thus, living and dead cells can be counted to a one-cell level by bonding of the first compound to nucleic acid, and hence, the living and dead cells can be counted precisely at a high sensitiveness even in the presence of impurities such as metabolites from microorganisms, dusts and inorganic substances.

The second compound is one capable of being bonded to nucleic acid. Thus, dead cells can be counted to a one-cell level by bonding of the second compound to nucleic acid, and hence, the dead cells can be counted precisely at a high sensitiveness even in the presence of impurities such as metabolites from microorganisms, dusts and inorganic substances.

The third compound is one for staining microorganisms by the reaction with a substance derived from microorganisms in the specimen, thereby providing an effect that the presence or absence of living microorganisms can be confirmed by the reaction of the third compound with a substance derived from microorganisms such as metabolites from microorganisms. In addition, the substance derived from microorganisms is an enzyme protein and thus, the presence or absence of living microorganisms can be judged at a higher sensitiveness.

The substance derived from particular microorganisms is an enzyme protein. In general, the enzyme protein is a substance resulting from the metabolism of living cells and hence, the living particular microorganisms can be counted without destruction of living cell membranes in a pretreatment.

In the microorganism counting process, a compound for staining particular microorganisms by the reaction with a substance derived from particular microorganisms is brought into contact with a specimen, and particular microorganisms are detected from a wavelength and an amount of color developed. Thus, a detecting reagent can be supplied in a suitable amount to the specimen, and the particular microorganisms can be counted at a high safety and a high sensitiveness without direct or indirect touching of an operator with the specimen.

### EXAMPLES

A microorganism detecting kit, a microorganism counting apparatus and a microorganism counting process according to the present invention will now be described in detail by way of embodiments, but the present invention is not limited in any way to the description below.

Example 1 of the present invention will be described below.

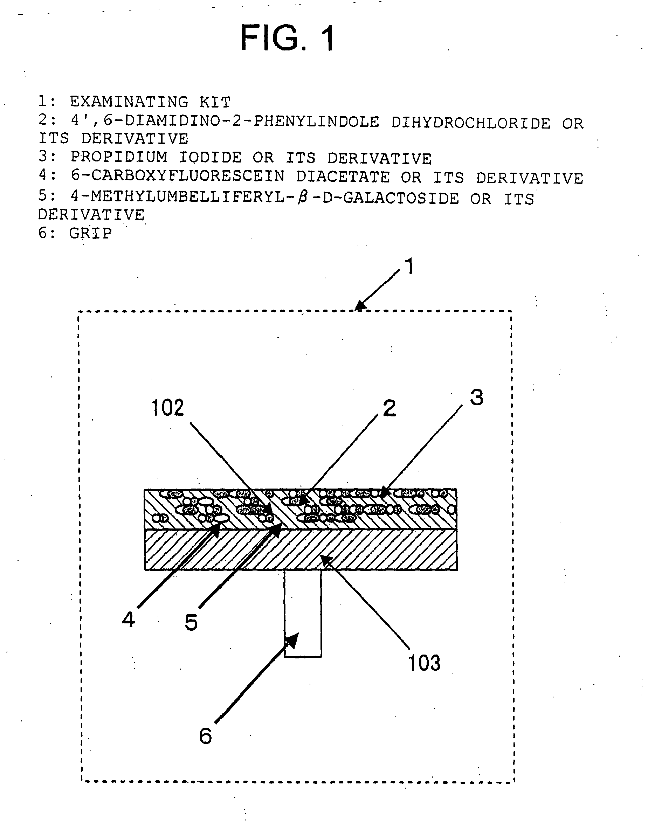

A microorganism detecting kit according to the present invention is shown in Fig. 1. Referring to Fig. 1, the detecting kit 1 is comprised of a self-adhesive layer 102 having the following compounds incorporated therein: 4',6-diamidino-2-phenylindole dihydrochloride (the derivative thereof may be used) 2 as a first compound for staining or coloring living and dead cells, propidium iodide (the derivative thereof may be used) 3 as a second compound for staining dead cells at a wavelength different from the above-described staining, 6-oarboxyfluoresoein diacetate (the derivative thereof may be used) 4 as a third compound for staining living cells at a wavelength different from the above-described staining, and 4-methylumbelliferyl-β-D-galactoside (the derivative thereof may be used) 5 as a fourth compound for staining particular microorganisms at a wavelength different from the above-described staining by reacting with a substance derived from particular microorganisms, a support 103, and a grip portion 6. The self-adhesive layer 102 includes an agar culture medium containing a meat essence, peptone and sodium chloride and agar, and is formed by mixing the compounds 2, 3, 4 and 5 together and solidifying the resulting mixture after being sterilized. In Fig.1, all of the compounds 2, 3, 4 and 5 are incorporated, but only one of the compounds may be incorporated, or two of the compounds may be incorporated, or three of the compounds may be incorporated.

In a process for examining cells or microorganisms, in order to deposit cells or microorganisms deposited to the specimen to the self-adhesive layer 102 including the agar culture medium containing the compounds, a rugged surface of a specimen and the self-adhesive layer 102 are pressed using the grip portion 6, whereby the specimen and the self-adhesive layer 102 including the agar culture medium are brought into close contact with each other by the fixed support 103.

After the cell or microorganisms deposited to the specimen are deposited to the self-adhesive layer 102 including the agar culture medium, the compounds 2, 3, 4 and 5 contained in the agar culture medium react with the cell or microorganisms to become individually bonded to the cell or microorganisms to stain or color them. Living and dead cells, dead cells and living cells, which exist, can be confirmed by counting the colored cells.

The 4',6-diamidino-2-phenylindole dihydrochloride for staining or coloring the living and dead cells and the propidium iodide for staining or coloring the dead cells are passed through cell membranes and bonded to nucleic acid.

The 4',6-diamidino-2-phenylindole dihydrochloride, after being bonded, absorbs exciting light at an exciting wavelength of 359 nm to emit light having a fluorescent wavelength of 461 nm to color the living and dead cells. The propidium iodide absorbs exciting light at an exciting wavelength of 535 nm to emit light having a fluorescent wavelength of 617 nm to color only the dead cells.

Further, the 6-oarboxyfluoresoein diacetate is decomposed by esterase produced by cells to produce a fluorescein, thereby emitting fluorescent rays as a result of application of exciting light.

The 4-methylumbelliferyl-β-D-galaotoside reacts with β-galaotosidase produced by Coliform bacteria to produce 4-methylumbelliferone, thereby emitting fluorescent rays as a result of application of exalting light.

In an actual use example, sterilized normal agar culture medium containing 4',6-diamidino-2-phenylindole dihydrochloride, propidium iodide, 6-oarboxyfluorescein diacetate and 4-methylumbelliferyl-p-D-galaotoside, which are detecting reagents, was to be formed a self-adhesive layer, and the self-adhesive layer was brought into direct contact with a wall surface of a drain tank in an air conditioner by using the grip and placed onto a microscope adapted to emit exciting light. Then, exciting light was applied to the surface of the self-adhesive layer. Cells irradiated by the exciting light are colored or stained at individual wavelengths, and the number of individual cells emitting fluorescent rays was counted visually through a light-separating film for separating the fluorescent rays. Germs were collected from the specimen and measured in 2 or 3 minutes. For comparison, microorganisms on the wall surface of the drain tank are collected and cultured in a standard agar food stamp (made by Nissui, Co.) for detecting common bacteria and in a deoxycholate food stamp (made by Nissui, Co.) for detecting Coliform bacteria, and the number of detected cells was counted. However, the food stamps could not detect living and dead cells and dead cells and could not culture thermophiles and special bacteria and for this reason, the number of cells detected was smaller than that detected by the microorganism detecting kit according to the present invention. The number of living Coliform bacteria detected was equal to that of living Coliform bacteria detected by using the microorganism detecting kit according to the present invention.

As described above, according to the present invention, amounts of rays emitted from the living and dead cells and the dead cells are counted from differences between wavelengths of colored- fluorescent rays and between amounts of rays emitted, thereby counting the numbers of the living cells and the dead cells. Further, the presence or absence of Coliform bacteria and living cells can be confirmed.

To count the deposited germs, they can be detected at 200 to 1, 000 magnifications by a magnifying lens such as a microscope.

The mixture after being sterilized was cooled and solidified to form the agar culture medium, but a liquid culture medium with agar removed can be used to confirm the presence or absence of living cells.

The Coliform bacteria were used as the particular microorganisms, but microorganisms to be detected may be those such as staphylococcus aureus and Vibrio parahaemolyticus.

The agar culture medium was used as a specimen contact means, but awater-soluble polymer such as gum arabic and gelatin, polyvinyl alcohol made by polymerizing a water-soluble monomer such as vinyl alcohol and acrylic acid, polyacrylic acid, and a polymer comprising two or more molecules of a water-soluble monomer can be used, if cells can be deposited thereto from a specimen.

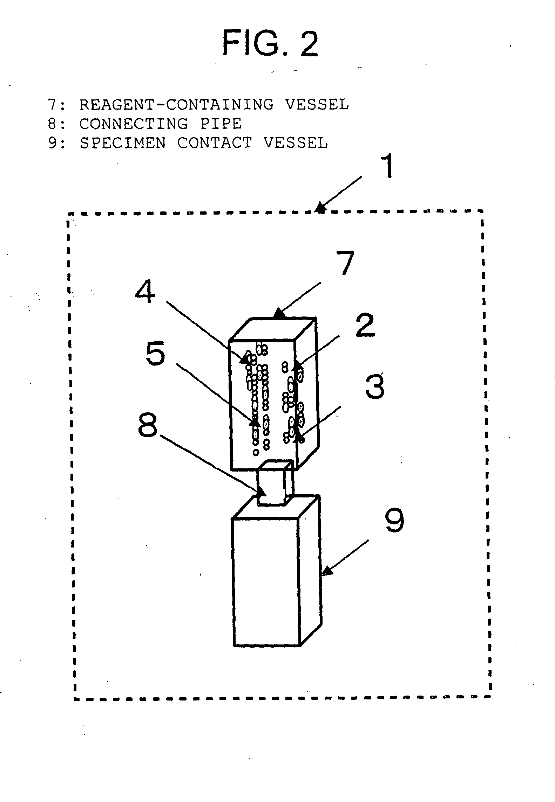

Example 2 of the present invention will be described below. An arrangement according to Example 2 is shown in Fig.2. This detecting kit 1 includes a reagent-containing vessel 7, into which the following compounds are incorporated: 4',6-diamldino-2-phenylindole dihydrochloride 2 as a first compound for staining or coloring living and dead cells, propidium iodide 3 as a second compound for staining dead cells at a wavelength different from the above-described staining, 6-carboxyfluoresceindiacetate 4 as a third compound for staining dead cells at a wavelength different from the above-described staining, and 4-methylumbelliferyl-β-D-galaotoside 5 capable of measuring Coliform bacteria as a fourth compound for staining particular microorganisms at a wavelength different from the above-described staining by reacting with a substance derived from particular microorganisms. The reagent-containing vessel 7 is constructed in such a manner it is connected to a specimen contact vessel 9 by a connecting pipe 8. Although not shown, an aqueous solution such as physiological saline or the like may be filled in the specimen contact vessel 9 in some oases. In Fig.2, all of the compounds 2, 3, 4 and 5 were incorporated, but only one of the compounds may be incorporated, or two of the compounds may be incorporated, or three of the compounds may be incorporated.

In the detecting kit 1, an aqueous solution containing cells or microorganisms or microorganisms collected from a specimen by an applicator or the like are poured into the specimen contact vessel 9, or dissolved into an aqueous solution within the specimen contact vessel 9. The 4',6-diamidino-2-phenylindole dihydrochloride 2, propidium iodide 3, 6-oarboxyfluorescein diacetate 4 and 4-methylumbelliferyl-β-D-galavtoside 5 capable of measuring Coliform bacteria 5 contained in the reagent-containing vessel 7 are mixed with cells contained in the specimen contact vessel 9 as a specimen contact means through the connecting pipe 8, and the numbers of dead cells, living and dead cells, living cells and particular microorganisms are counted from colors developed by the reagents.

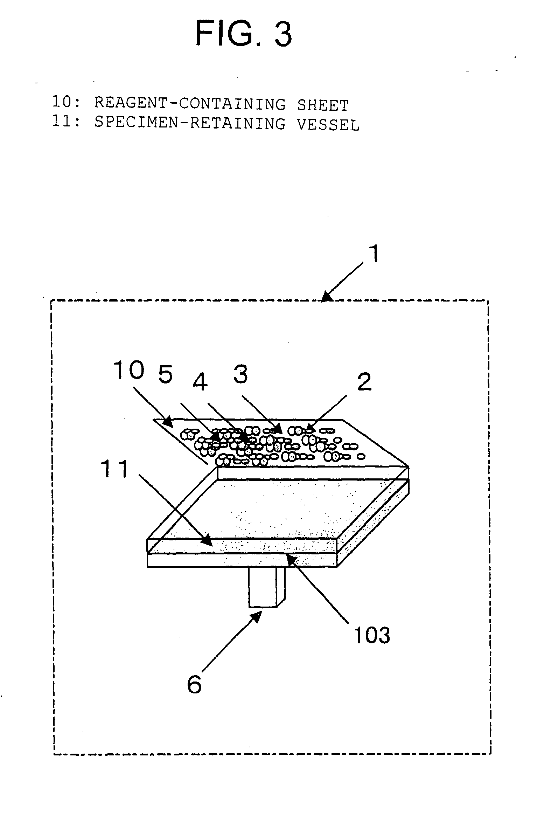

Even if a detecting kit is formed by using a reagent-containing sheet 10 in place of the reagent-containing vessel and using a specimen-retaining vessel 11 in place of the specimen contact vessel, as shown in Fig.3, a similar effect is provided.

In an actual use example, a detecting kit was used, which was formed using a normal agar culture medium as a specimen contact means, and using a detecting-reagent containing section formed by a film retaining a solution containing 4',6-diamidino-2-phenylindole dihydrochloride, propidium iodide, 6-carboxyfluorescein diacetate and 4-methylumbelliferyl-P-D-galaotoside. The normal agar culture medium as the specimen contact means was brought into contact with a wall surface of a drain tank in an air conditioner and after the contact, the film containing the detecting reagents was brought into close contact with the surface of normal agar culture medium in contact with the wall surface. The specimen contact means and the detecting-reagent containing section left in contact with each other were placed onto a microscope capable of emitting exciting light, and exciting light was applied to them. Cells irradiated by the exciting light were stained or colored at individual wavelengths, and the number of individual cells emitting fluorescent rays was counted visually through a light-separating film for separating fluorescent rays. Germs were collected from the specimen and measured in 2 or 3 minutes. For comparison, microorganisms on the wall surface of the drain tank are collected and cultured in a standard agar food stamp (made by Nissui, Co.) for detecting common bacteria and in a deoxycholate food stamp (made by Nissui, Co.) for detecting Coliform bacteria, and the number of detected cells was counted. However, the food stamps could not detect living and dead cells and dead cells and could not culture thermophiles and special bacteria and for this reason, the number of cells detected was smaller than that detected by the microorganism detecting kit according to the present invention. The number of living Coliform bacteria detected was equal to that of living Coliform bacteria detected by using the microorganism detecting kit according to the present invention.

With the above-described arrangement, the movement of the compound to the specimen due to the direct contact of the compound to the specimen can be prevented, and microorganisms existing in the specimen can be detected.

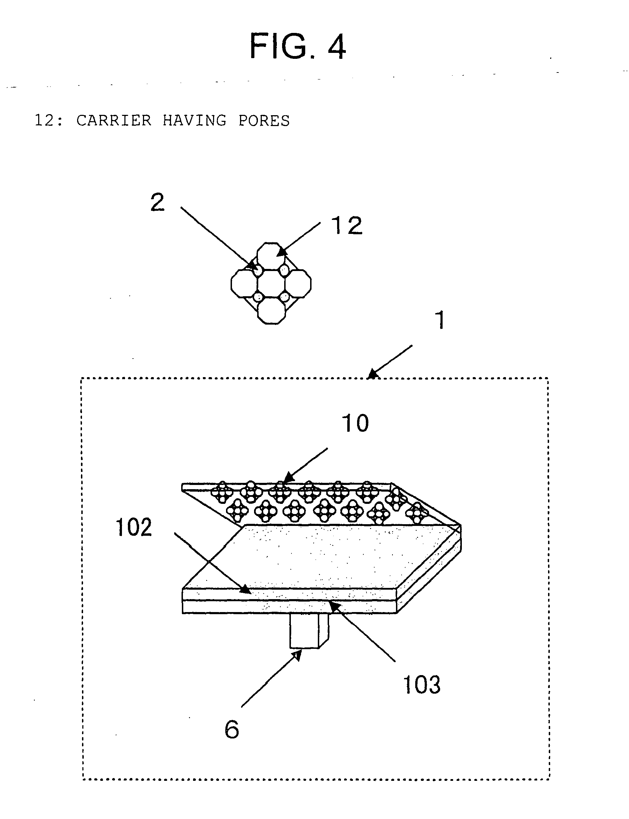

Example 3 of the present invention will be described below. An arrangement is shown in Fig.4. A detecting kit 1 in Example 3 is comprised of a reagent-containing sheet 10 retaining, on its surface, carriers 12 made by using activated carbon as a starting material and each providing with pores and each having 4',6-diamidino-2-phenylindole dihydrochloride 2 supported thereon, a self-adhesive layer 102 which is a specimen contact means, a support 103 retaining the self-adhesive layer 102, and a grip 6 for bringing a specimen into close contact with the self-adhesive layer 102.

With the above-described arrangement, the 4',6-diamidino-2-phenylindole dihydrochloride 2 supported in the pores in the carriers 12 is protected from the oxidation due to a warm heat provided by the natural leaving, ultraviolet rays contained in natural light, or air, and the detecting kit 1 can be preserved for a long time.

It should be noted that by supporting, on a carrier having pores such as the carriers 12 having the pores, propidium iodide as the second compound for staining the dead cells at the wavelength different from the above-described staining, 6-carboxyfluorescein diacetate as the third compound for staining the living cells at the wavelength different from the above-described staining, and 4-methylumbelliferyl-β-D-galaotoside capable of measuring Coliform bacteria, as the fourth compound for stating the particular microorganisms at the wavelength different from the above-described staining by the reaction with the substance derived from particular microorganisms, the preservation of them can be facilitated, and the detecting sensitiveness can be enhanced by an Increase in area of contact with the specimen.

In the example, the 4',6-diamidino-2-phenylindole dihydrochloride was supported on the carriers 12 made by using activated carbon as the starting material and having the pores, but even if propidium iodide, 6-carboxyfluoresceln diacetate and 4-methylumbelliferyl-β-D-galactoside capable of measuring Coliform bacteria may be supported in the form of a mixture on activated carbon 10, a similar effect can be provided.

The carrier 12 is a carrier having pores, but if the carrier is one having pores such as zeolite and silica gel, a similar effect can be provided.

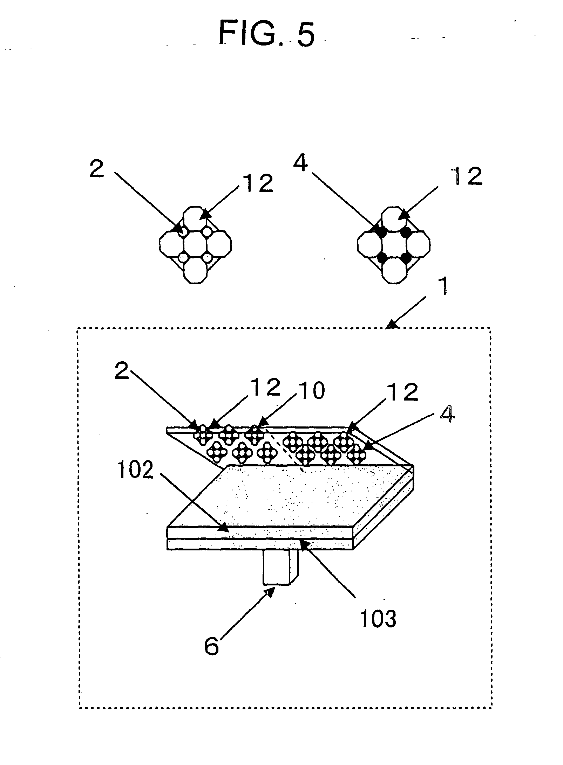

Example 4 of the present invention will be described below. An arrangement is shown in Fig.5.

A detecting kit 1 in Example 4 is comprised of a reagent-retaining layer 10 retaining, in a separated manner on its surface, carriers 12 made by using activated carbon as a -starting material and each providing with pores and each having 4',6-diamidino-2-phenylindole dihydrochloride 2 supported thereon as a first compound for staining living and dead cells, and carriers 12 made by using activated carbon as a starting material and each providing with pores and each having 6-carboxyfluoresoein diacetate 4 supported thereon as a second compound for stating living cells, a self-adhesive layer 102, a support 103 retaining the self-adhesive layer 102, and a grip 6 for bringing a specimen into close contact with the self-adhesive layer 102.

With the above arrangement, the fluorescent reagents are moved from the carriers 12 contained on the reagent-retaining layer 10 to living and dead cells or dead cells, or to Coliform bacteria or living cells, which is deposited to the self-adhesive layer 102 from the specimen, to dye or stain the various cells. In this case, the reagent-retaining layer 10 is bisected, and the carriers 12 retaining the reagents in the above combination are retained in the resulting separated sections of the reagent-retaining layer 10. To carry out the detection, light having a wavelength peculiar to each of the fluorescent reagents is applied to each of the separated sections to stain intended cells.

Thus, it is possible to detect the presence or absence of Coliform bacteria, the number of all germs, the number of living germs and the number of dead germs at one time and to grasp the state of the specimens in a short time.

It should be noted that the carrier is not limited to the activated carbon.

In addition, if even one type of the reagent is supported on the carrier, a useful effect can be provided.

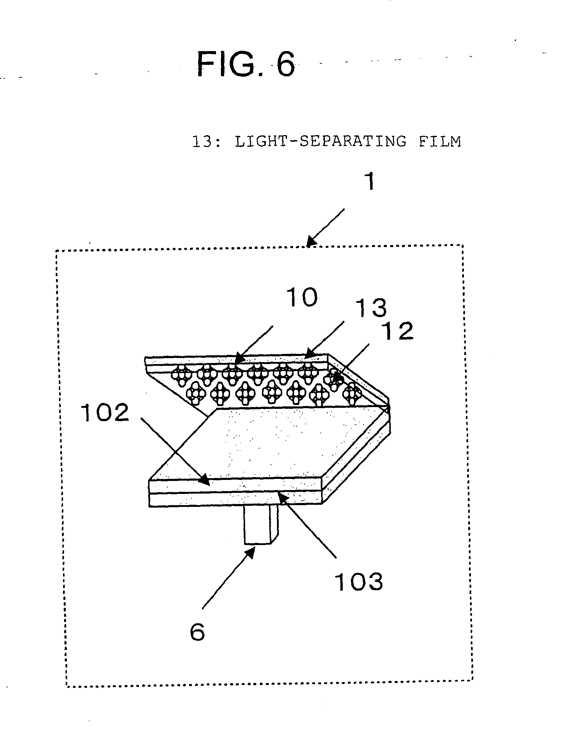

Example 5 of the present invention will be described below. An arrangement is shown in Fig.6.

A detecting kit 1 in Example 5 is comprised of a self-adhesive layer 102 adapted to be brought into direct contact with a specimen to collect cells deposited to the specimen, a support 103 for supporting the self-adhesive layer 102, a grip 6 for enhancing the operability of the detecting kit, carriers 12 containing a reagent colored by the presence of cells in pores, a reagent-containing sheet 10 for retaining the carriers 12, and a light-separating film 13 for separating applied exciting light into light having an intended wavelength.

With the above arrangement, living and dead cells, dead cells and particular microorganisms deposited to the self-adhesive layer 102 are stained or dyed by the staining reagents supported in the carriers 12, and exciting light is applied through the light-separating film 13 to the stained cells and microorganisms. The exciting light stains the cells deposited to the self-adhesive layer 102 through the light-separating film 13 and hence, even if the exciting light is a light of a wider wavelength emitted from a light source, the cells can be detected and counted only by the detecting kit.

The light-separating film 13 is not only available to separate the exciting light but also available to separate fluorescent rays.

The light-separating film 13 may be comprised a film for separating the exciting light and a film for separating the fluorescent rays, which are superposed onto each other. In this case, the separation of the exciting light and the separation of the fluorescent rays produced by the exciting light can be carried out at one time.

The light-separating film 13 may be mounted on the support 103.

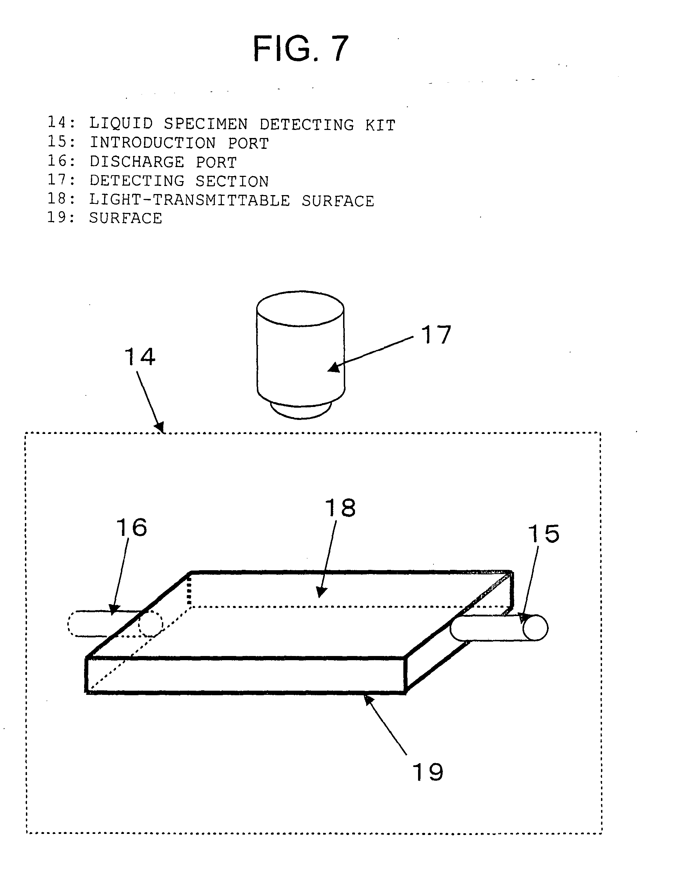

Example 6 of the present invention will be described below. An arrangement is shown in Fig.7.

A liquid specimen detecting kit 14 in Example 6 includes an introduction port 15 for throwing a liquid specimen, and a discharge port 16 for discharging a surplus amount of liquid specimen and air previously contained in the liquid specimen detecting kit 14. The liquid specimen detecting kit 14 is of a structure in which a surface, on which at least a detecting section 17 exists, is a light-transmittable surface 18, so that microorganisms can be detected in the detecting section. Further, a surface 19 opposed to the light-transmittable surface 18 has a structure in which it is in a parallel-positional relationship to the light-transmittable surface 18 and it is not inclined.

With the above arrangement, even if the specimen is liquid, it can be retained in a given space. In addition, when microorganisms contained in the liquid specimen have been introduced into the liquid specimen detecting kit 14, the microorganisms can be diffused in a given region without being superposed one on another, because the distance between the light-transmittable surface 18 and the surface 19 located in an opposed relation to the surface 18 are maintained at a given thickness. Thus, it is possible to prevent the underestimation due to the superposition and to count the microorganisms precisely by a detecting means placed on the detecting section 17.

It should be noted that the sensitiveness for detecting microorganisms can be enhanced by mounting a filter in front of the introduction port 15 in order to remove foreign matters.

In addition, the light-transmittable surface 18 can be formed so that it does not influence the exciting light such as ultraviolet rays, thereby preventing the obstruction of the exciting light.

The distance between the light -transmittable surface 18 and the surface 19 located in an opposed relation to the surface 18 is in a range of 0.5 to 15 µm. Thus, it is possible to prevent the superposition of microorganisms and to achieve the precise discrimination.

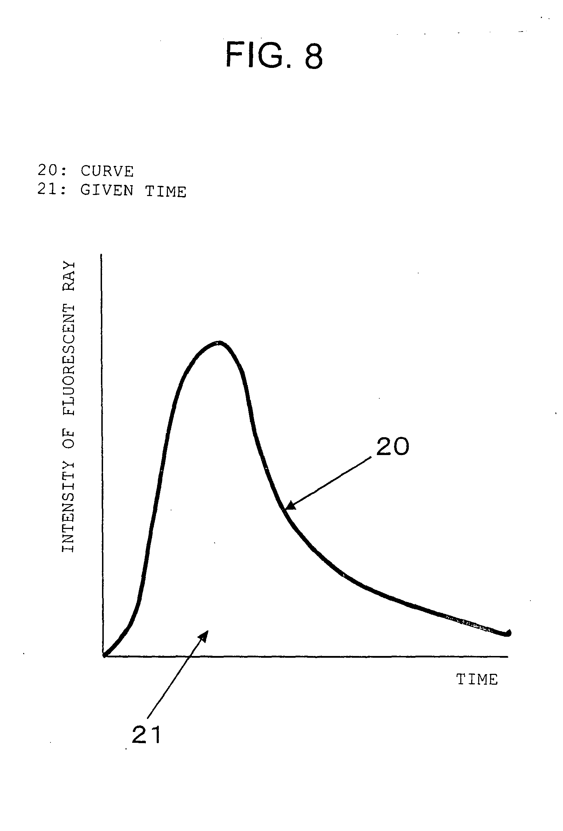

Example 7 of the present invention will be described below. Fig.8 is a curve of attenuation of fluorescent light.

When exciting light is continuously applied to a fluorescent coloring matter, the intensity of fluorescent light emitted by the exciting light forms a curve of attenuation as shown in the curve 20 due to the fading. A given time 21 for maintaining the intensity including and around the maximum intensity of fluorescent light existing in the curve of attenuation is provided in Fig.8.

With the above arrangement, for the intensity of fluorescent light attenuated as shown in the curve 20, even when counting after attenuation and not being counted, the given time 21 for maintaining the intensity including and around the maximum intensity of fluorescent light is provided, and the addition and counting are carried out within such time, whereby the mis-recognition due to the fading of the fluorescent coloring matter can be prevented to enable the precise counting.

The given time 21 can be set to be peculiar to the coloring matter, and may be changed depending on the type of the coloring matter.

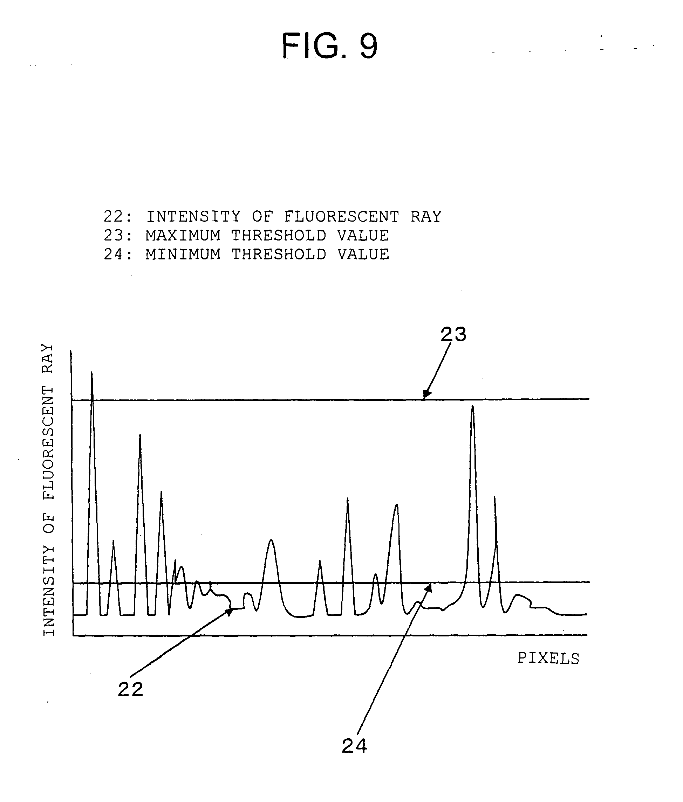

Example 8 of the present invention will be described below. Fig.9 is a diagram indicating threshold values with respect to the intensity of fluorescent light.

When microorganisms existing on a specimen contact means and stained or colored to emit rays have been detected, an intensity 22 of fluorescent rays as shown in Fig. 9 can be obtained. A maximum threshold value 23 and a minimum threshold value 24 derived from the intensity of fluorescent rays of microorganisms are provided for the intensity 22, and values of intensity 22 of fluorescent rays existing between the maximum and minimum threshold values 23 and 24 are recognized as fluorescent rays derived from microorganisms and added all together to count the microorganisms.

With the above arrangement, peaks having intensity values of fluorescent rays existing between the maximum and minimum threshold values 23 and 24 derived from the intensity of fluorescent rays of the microorganisms are recognized as microorganisms, and other values are determined as provided by foreign matters and are not counted, thereby enabling the precise counting.

The threshold values may be varied depending on the coloring matter, and a preset time suitable to the varied values may be provided.

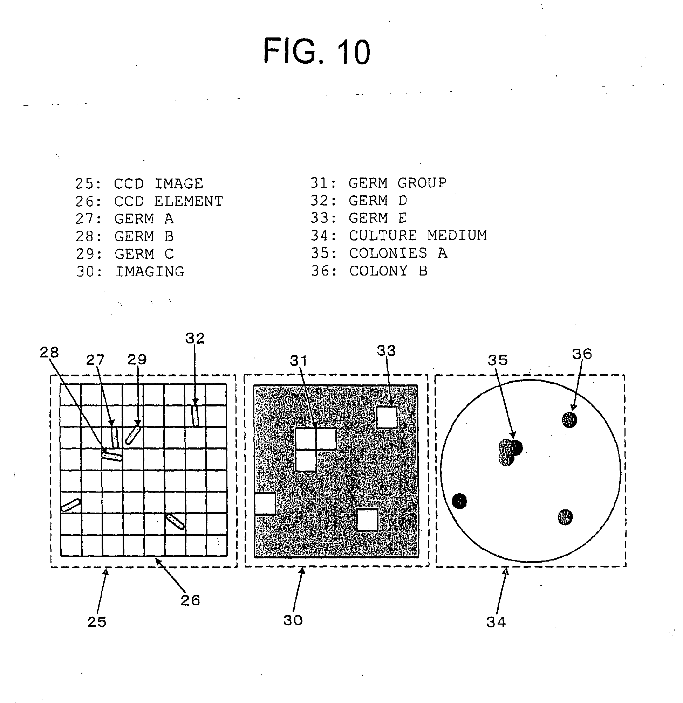

Example 9 of the present invention will be described below. An arrangement is shown in Fig.10.

Microorganisms existing on a specimen contact means and colored to emit light are shown in a CCD image 25. When they are recognized by a CCD element 26, rays emitted from microorganisms are recognized within pixels. In this case, when a pixel with a germ B28 recognized therein and a pixel with a germ C29 recognized therein exist around a pixel with a germ A27 recognized, the germ A27, the germ B28 and the germ C29 are recognized as a germ group 31, as shown in an imaging diagram 30. When a germ such as a germ D32 is recognized within one pixel, it is recognized as one germ E33, as shown in the imaging diagram 30, and the germs in the pixels are counted in an adding manner. When the germs existing at locations on the CCD image 25 have been cultured, colonies A35 and a colony B36 as shown in a culture medium 34 are formed.

With the above arrangement, when a large number of germs exist, adjacent germs are recognized as one germ group 31 and counted as one bright point in an adding manner. Thus, when the counting of the germs are carried out in the culture process which is one of the prior art processes is carried out, the correlation to the prior art process can be provided in consideration of the fact that the germ group is recognized as one colony.

Example 10 of the present invention will be described below.

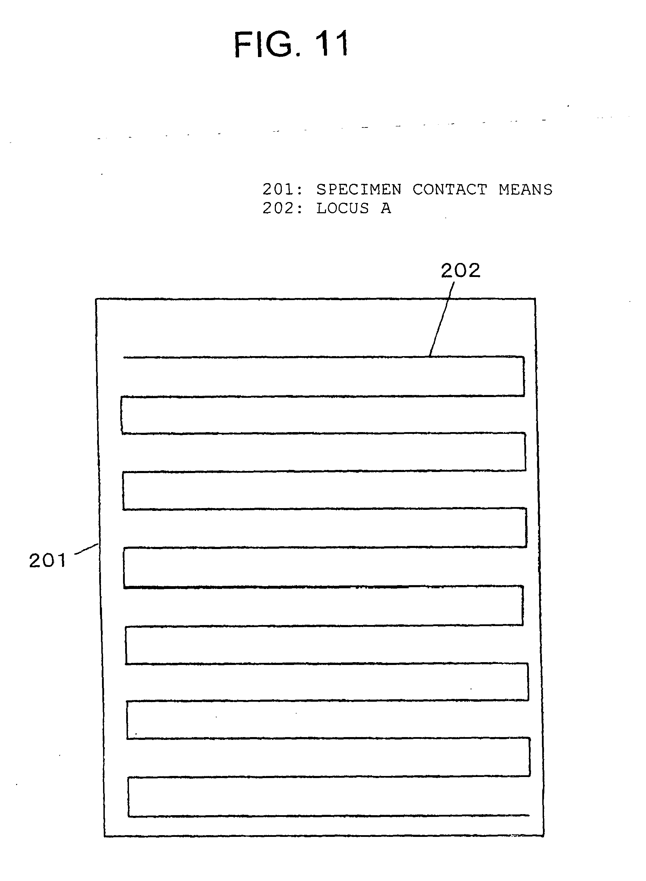

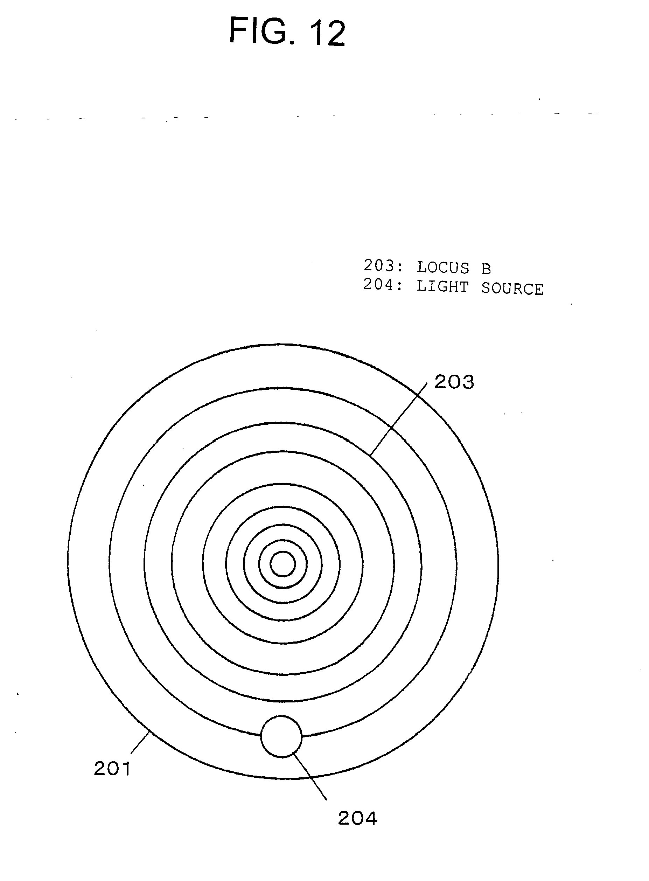

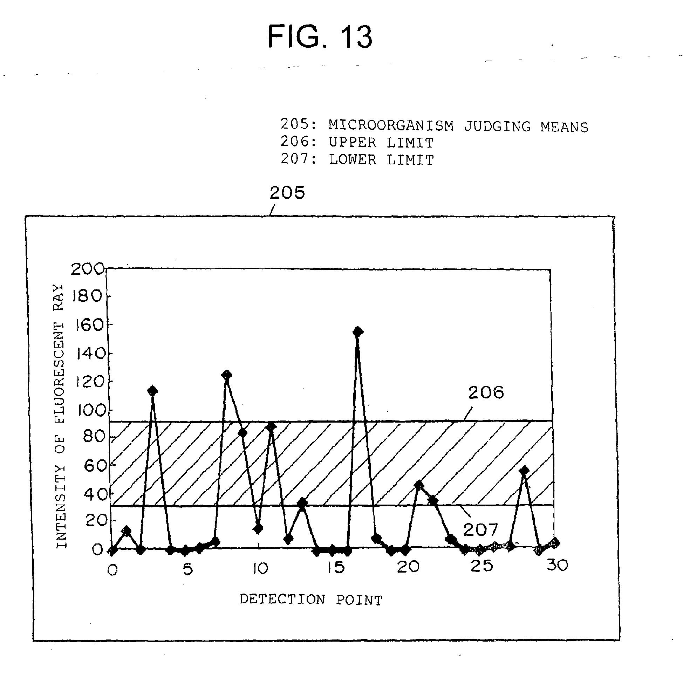

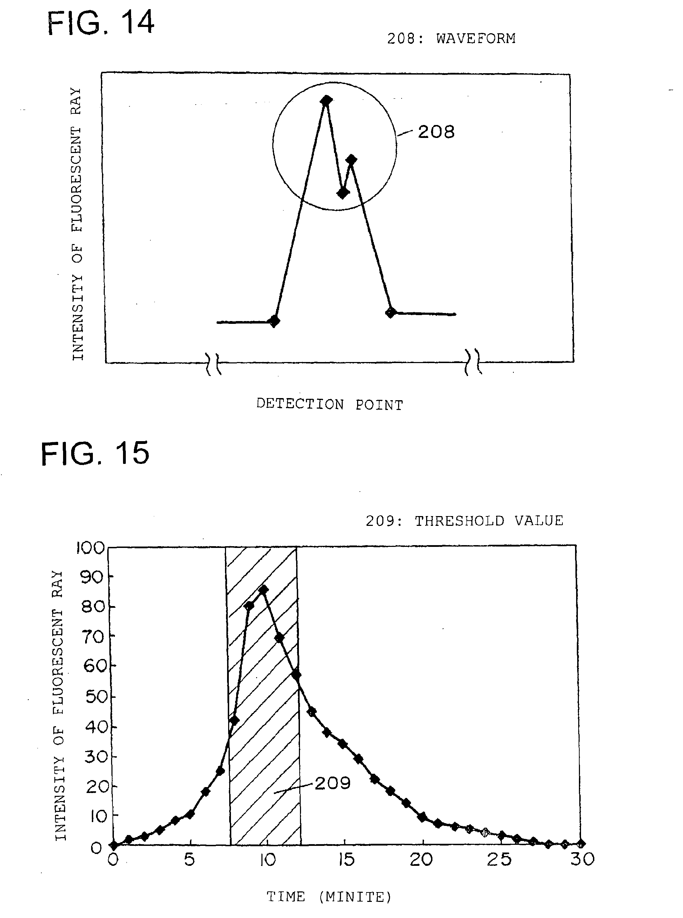

It is desired that a locus of exciting light on a specimen placed on a microorganism counting apparatus, as shown in Fig.11, covers the entire specimen contact means 201. For this purpose, the exciting light is moved for detection as along a locus A202 to cover the width and the length of a specimen. The locus for detecting the entire specimen may be circular as shown in Fig.12. Therefore, the exciting light can be moved as along a locus B203 to detect microorganisms. Shown in Fig.13 is a model of a microorganism judging means in which a light source 204 is moved along each of the loci on the specimen contact means, and using fluorescent rays produced by the exciting light emitted from the light source 204. Fluorescent rays emitted from a specimen forms a microorganism judging means 205 in correlation to sites emitting the fluorescent rays. Scattered rays produced from impurities or the like, fluorescent rays generated by the exciting light and the like coexist on the microorganism judging means 205, and rays are not same as fluorescent rays emitted from target microorganisms in some oases. Therefore, threshold values are provided. The threshold values include an upper limit value 206 and a lower limit value 207 for the intensity of fluorescent rays, and fluorescent rays within a range between the upper and lower limit values are recognized as derived from microorganisms and added all together. Thus, fluorescent rays derived from impurities other than microorganisms can be grasped to enable the precise counting of microorganisms. The threshold values are determined by the intensity of fluorescent rays emitted by microorganisms. When adjacent microorganisms exist, the waveform provided by fluorescent rays emitted from microorganisms is of a profile indicating the existence of the adjacent microorganisms as shown in Fig. 14. In this case, when the adjacent microorganisms have been detected in an agar culture medium diffusion process which is one of the prior art processes, colonies may be superposed one on another with the propagation of the microorganisms and the coalescence of colonies and determined as one colony at a time point of confirmation visually in some oases. Therefore, according to the present invention, in a case as shown in the waveform 208, the adjacent microorganisms are detected as one microorganism. Thus, it is possible to provide the correlation to the agar culture medium diffusion process.

The time of application of exciting light to the specimen contact means 201 is set in a range in which the intensity of fluorescent rays is not extinguished, as shown in Fig.15. Namely, the time is in a range of threshold values 209.

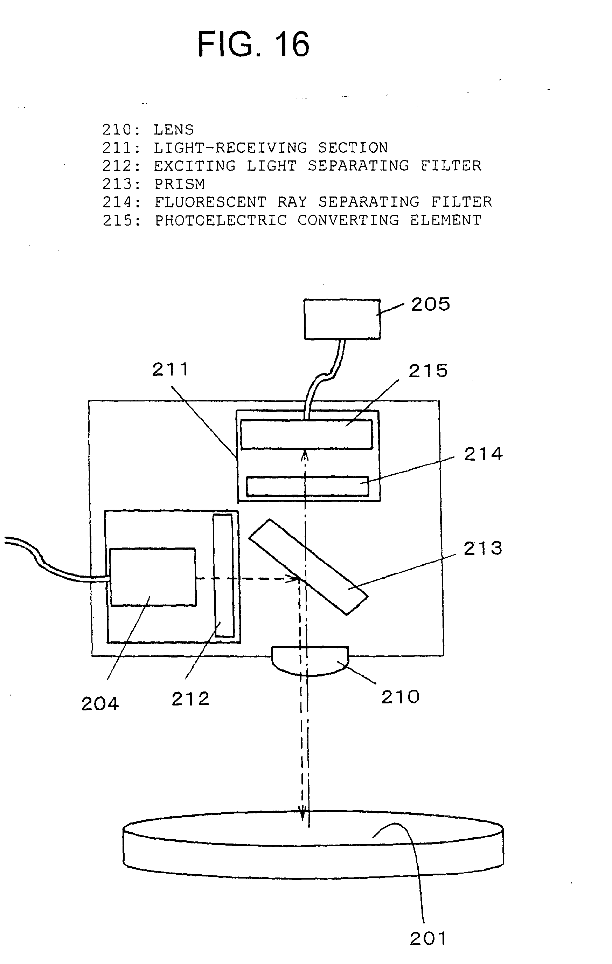

As shown in Fig.16, the microorganism counting apparatus comprises a light source 204, a lens 210 as a light collector means, and a light-receiving section 211. Exciting light emitted from the light source 204 is separated by an exciting light separating filter 212 to extract a target wavelength. The separated exciting light is diverted through a prism 213 into a path. The diverted exciting light is passed through the lens 210 and collected onto the surface of a specimen contact means 201. Fluorescent rays emitted by microorganisms excited on such surface by the exciting light are transmitted again through the prism 213. In this case, the fluorescent rays are transmitted, as they are, through the prism 213 to reach the light-receiving section 211. The fluorescent rays reaching the light-receiving section 211 are passed through a fluorescent ray separating filter 214 to extract only a target fluorescent ray, and to reach a photoelectric converting element 215 included in the light-receiving section 211, where they are converted into signals and recognized. Although not shown, the specimen contact means 201 or the microorganism counting apparatus is provided with a means for moving the specimen contact means, so that all or some of fluorescent colors developed in the specimen contact means 201 can be received.

The fluorescent rays reaching the photoelectric converting element 215 are judged as derived from microorganisms or foreign matters in the microorganism judging means 205. The fluorescent rays judged as derived from microorganisms are added all together, and the number of microorganisms is counted.

The exciting light emitted from the light source 204 is collected by the lens 210, and the exciting light to be applied by the lens 210 is collected in a region of a very small given area. In this case, the term "very small given area" indicates a region on the order of 0.2 to 7.0 µm per side, as determined based on the size of bacteria or true fungi. On the basis of comparison with the agar culture medium diffusion process which is one of microorganism detecting means utilized currently in most cases, colonies formed by the group of microorganisms cultured and propagated by the agar culture medium diffusion process may be superposed one on another if they are closer in distance to one another and finally, the superposed colonies may be recognized as one colony as confirmed visually in some oases. In this case, the term "very small given area" indicates a region on the order of 100 to 500 µm per side, when it is based on the distance between the non-superposed colonies.

The time of application of the exciting light collected by the lens 210 depends on the time of extinction of the compound emitting fluorescent light and the intensity of the exciting light. Depending on the type of the compound, the compound may be decomposed even by ultraviolet rays existing in the natural world and hence, it is desirable that the exciting light is applied for a time in a range of about 2 second to about 300 seconds.

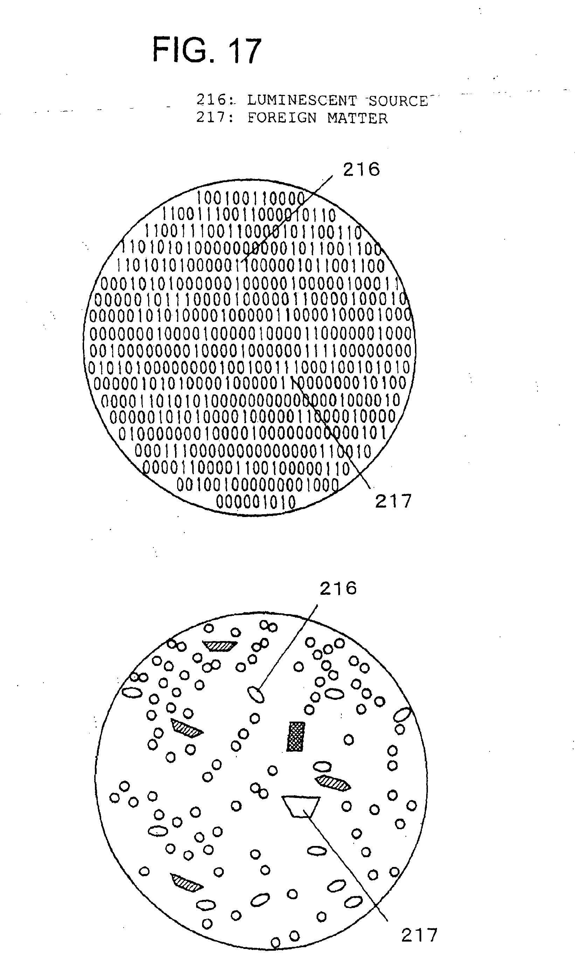

When the luminance after the coloration is recognized, a position of fluorescent coloration is represented by a binary system comprising "0" and "1", as shown in Fig.17. In the binary system, adjacent colors developed are regarded as one luminescent source 216. If the size and the luminance of the luminescent source are relatively largely different from preset values, such luminescent source is recognized as a foreign matter 217 and regarded as out of the examination and is not counted as microorganism. As for a distance between adjacent colors developed, on the basis of comparison with the agar culture medium diffusion process which is one of microorganism detecting means utilized currently in most oases, colonies formed by the group of microorganisms cultured and propagated by the agar culture medium diffusion process may be superposed one on another if they are closer in distance to one another and finally, the superposed colonies may be recognized as one colony as confirmed visually in some oases. Thus, the term "very small given area" indicates a region on the order of 100 to 500 µm per side, when it is based on the distance between the non-superposed colonies.

Microorganisms deposited on a specimen emit fluorescent rays by virtue of the coloring compound. Typical of the coloring compound are 4',6-diamidino-2-phenylindole, which is one of reagents capable of being permeated non-specifically into microorganism cells irrespective of life or death of the microorganisms and bonded to nucleic acid in the microorganisms to color the microorganism cells, and propidium iodide which is one of reagents capable of being permeated non-specifically into dead cells and bonded to nucleic acid in the microorganisms to color the microorganism cells. Examples of other coloring reagents are 6-oarboxyfluoresoein diacetate, 2',7'dichlorofluorescein diacetate, 6-(N-succinimidyloxyoarbonyl)-3',6'-0,0'-diacetylfluoresoei n, dihydrodorhodamine, fluorescein diacetate, 4-azidofluorescein diacetate and the like. The luminescence relies on the activity of an enzyme in the cells and is capable of dyeing living germs. Examples of reagents for dyeing particular microorganisms such as Coliform bacteria are 4-methylumbelliferyl-β-D-galactoside, 4-methylumbelliferyl-β-D-glucuronide and the like. These compounds react specifically with an enzyme produced by particular microorganisms to become decomposed into 4-methylumbelliferron. The 4-methylumbelliferron is excited by ultraviolet rays to emit fluorescent light. Thus, the number or the presence or absence of particular microorganisms can be detected by detecting the fluorescent light emitted by the excitation by the ultraviolet rays.

The present invention provides a means for detecting microorganisms after being colored or dyed. The compounds have different maximum exciting wavelengths and emit fluorescent rays having different wavelengths, after being excited, thereby enabling the multiplex dyeing. In the detection of colors developed, if the light from the light source 204 has a wider wavelength, the exciting light can be regulated in wavelength and separated by the exciting light separating filter 212. The exciting light separating filter 212 can be exchanged depending on a target subject to be detected and hence, it is possible to accommodate various fluorescent reagents. At the same time, if the fluorescent colored light has a wider wavelength, it is possible to accommodate various fluorescent reagents by exchanging the fluorescent light separating filter 214 depending on a target subject to be detected in order to detect a target color developed.

Examples of the light source 204 are various diodes, a halogen lamp, a xenon lamp, a cold cathode tube, a laser, a black light, a mercury lamp and the like. Among these light sources, any of the diode, the cold cathode tube and the black light with the maximum exciting wavelength limited relatively can be mounted without use of the exciting light separating filter 212 and the fluorescent light separating filter 214 in some oases. When the halogen lamp, the mercury lamp or the like is used, the use of the exciting light separating filter 212 and the fluorescent light separating filter 214 may be required in some cases.

The prism 213 and the lens 210 have a nature that they transmit ultraviolet rays, as required. Examples of materials having the ultraviolet ray-transmittable nature for forming the prism 213 and the lens 210 are a quartz glass and the like. Thus, it is possible to accommodate fluorescent reagents excited by ultraviolet rays. The specimen contact means 201 may be placed on a member which is rotatable. The exciting light collected by the lens 210 is moved from an outer peripheral portion to a center portion of the specimen contact means 201, or from a center portion to an outer peripheral portion of the specimen contact means 201 through a distance corresponding to the radius of specimen contact means 201. In this case, it is possible to prevent the occurrences of the slippage, the persistence of vision and the afterglow of the fluorescent light emitted by the excited compound when the exciting light collected by the lens 210 exists at the outer peripheral portion and when the exciting light collected by the lens 210 exists at the center portion, by changing the rotating speed of the specimen contact means 201 when the position of the exciting light collected by the lens 210 is at the outer peripheral portion and when the position of the exciting light collected by the lens 210 is at the center portion.

A means is provided for recognizing the position in which the exciting light has been collected. Thus, the position of the exciting light collected by the lens 210 is recognized, so that the collection of the light is prevented from being deviated from the locus, and when the collection of the light has been deviated, it is returned again to the locus.

The coloring or staining reagents having the cell permeability have been exemplified, but any of coloring or staining reagents may be used which have a cell membrane depositability, a cell wall depositability, a sugar chain bondability, a functional group bondability, an amino acid adherability, an ion sensitivity, an oxygen reactivity, and even in this case, cells can be likewise detected.

In addition, the coloring or staining reagents excited by the exalting light to emit the fluorescent light has been exemplified, but any of coloring or staining reagents having an ability of staining and coloring cells may be used and even in this case, cells can be likewise detected.

The very small given area, to which the exciting light is applied, is not limited to the polygonal shapes including a square shape, and may be circular, elliptic or the like, if a specimen can be irradiated.

The compounds contained in the specimen contact means are capable of carrying out the multiplex dyeing, but they are not necessarily required to be mixed together. For example, a single specimen contact means maybe divided into portions corresponding to the number of dyeing compounds, so that cells can be detected in such portions, respectively.

A diffraction grating may be utilized as a means for separating the exciting light or the fluorescent light.

The persistence of vision and the afterglow of the fluorescent light are prevented by adjusting the rotating speed of the specimen contact means 201, but may be prevented by adjusting the moving speed of the lens 210 for applying the exciting light.

The means for recognizing the light collection position is not necessarily required to directly recognize the exciting light collection position, and may be a means capable of grasping the locus on the specimen contact means 201.

Example 11 of the present invention will be described below.

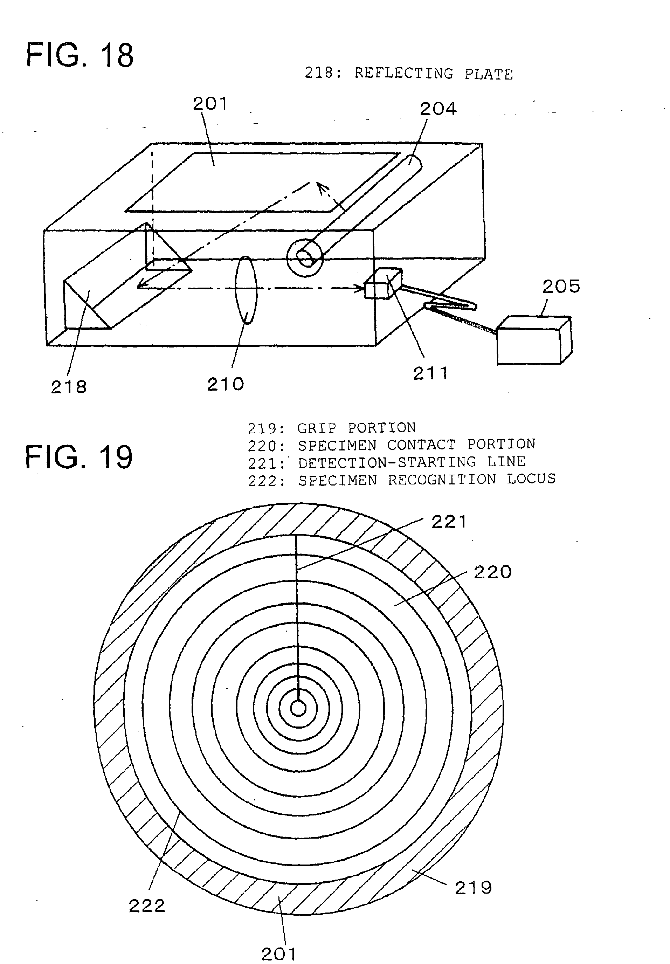

As shown in Fig.18, a microorganism counting apparatus comprises a specimen contact means 201, a light source 204, a reflecting plate 218, a lens 210, and a light-receiving section 211. Fluorescent rays excited and produced from dyed microorganisms existing in a specimen by the exiting light emitted from the light source 204 are turned in direction by the reflecting plate 218, collected by the lens 210 and detected by the light-receiving section 211. The fluorescent rays reaching the light-receiving section 211 are judged as microorganisms or foreign matters in a microorganism judging means 205. The fluorescent rays judged as the microorganisms are added all together, whereby the number of the microorganisms is counted. The light source 204 scans the specimen contact means 201. Further, the reflecting plate 218 is placed,in correspondence to the locus of the light source 204.

The exciting light emitted from the light source 204 is 8 collected by the lens 210, and the exciting light to be applied is collected in a region of a very small given area. In this case, the term "very small given area" indicates a region on the order of 0.2 to 7.0 µm per side, as determined based on the size of microorganisms such as bacteria. On the basis of 13 comparison with the agar culture medium diffusion process which is one of microorganism detecting means utilized currently in most cases, colonies formed by the group of microorganisms cultured and propagated by the agar culture medium diffusion process may be superposed one on another if they are closer in distance to one another and finally, the superposed colonies may be recognized as one colony as confirmed visually in some oases. In this case, the term "very small given area" indicates a region on the order of 100 to 500 µm per side, when it is based on the distance between the non-superposed colonies.

The time of application of the exciting light collected by the lens 210 depends on the time of extinction of the compound emitting fluorescent light and the intensity of the exciting light. Depending on the type of the compound, the compound may be decomposed even by ultraviolet rays existing in the natural world and hence, it is desirable that the exciting light is applied for a time in a range of about 2 second to about 300 seconds.

Examples of the light source 204 are various diodes, a halogen lamp, a mercury lamp, a xenon lamp, a cold cathode tube, a laser, a black light and the like.

The reflecting plate 218 and the lens 210 have a nature that they transmit ultraviolet rays, as required. Examples of materials having the ultraviolet ray-transmittable nature for forming the reflecting plate 218 and the lens 210 are a quartz glass and the like. Thus, it is possible to accommodate fluorescent reagents excited by ultraviolet rays.

In order to prevent any influence to the fluorescent light due to a reduction in intensity of the exciting light emitted from the light source 204 to the specimen contact means 201 or due to external light, a dark-field view plate or the like for placing the specimen contact means 201 in a dark-field environment may be placed.

The light source 204 is not necessarily movable. It is possible to detect the deposited microorganisms by moving the specimen contact means 201.

The light source 204 is not necessarily built in the microorganism counting apparatus. A light source section including a light source 204 may be mounted outside the apparatus, so that exciting light can be introduced from the light source section into the apparatus utilizing an optical fiber or the like.

A means for removing lights of wavelengths other than a target wavelength may be provided depending on the type of the light source. Examples of such means are an exciting light separating filter, a diffraction grating and the like.

In addition, a means for removing lights of wavelengths other than a target wavelength may be provided depending on the type of fluorescent light emitted. Examples of such means are a fluorescent light separating filter, a diffraction grating and the like.

Further, a prism may be utilized in place of the reflecting plate 218.

Example 12 of the present invention will be described below.

As shown in Fig. 19, a specimen contact means 201 comprises a grip portion 219 for enhancing the handleability for an operator, a specimen contact portion 220 for deposition of microorganisms from s subject of detection, a detection-starting line 221 for recognizing a detection-starting point, and a specimen recognition locus 222 for recognizing a detecting locus.

An operator takes a grip portion 219 by his hand and pushes it against a target subject of examination for several seconds. At this time, the pushing is carried out at a strength and for a time as much as the specimen contact portion 220 is not crashed. Microorganisms deposited from the subject of examination are dyed by the addition of a compound for staining or dyeing microorganisms depending on the state or nature of the microorganisms or by the permeation of a compound previously contained in the specimen contact portion 220. The detection of the specimen contact means 201 containing the dyed microorganisms is started. The specimen recognition locus 222 previously provided in the specimen contact means 201 to recognize the position of the exciting light collected on the specimen contact means 201 is detected to recognize the position of the exciting light. The exciting light collected by the lens 210 shown in Fig.16 is moved around an outer periphery from a start point on the detection-starting line 221. After movement of the exciting light around the outer periphery, when the exciting light again reaches the detection-starting line 221, the exiting light collected by the lens 210 shown in Fig.16. is diverted into a next inner portion of the specimen recognition locus 222, and thus, the number of colors included in such inner locus portion is recognized. Such a movement is repeated, namely, the exciting light is moved toward the center, whereby the number of microorganisms included in an area where the detection is required is detected.

It is desirable that a substance used in the specimen contact portion 220 is one which does not influence fluorescent light. It is also desirable to use a substance having a self-adherent property in order to efficiently bring microorganisms into contact with the specimen contact portion 220 from a specimen. Examples of such substances are various polymers such as gum arabic, acrylic resins and the like, substances containing moisture moderately and having a self-adherent property, such as agar, and the like.

Examples of the detection-starting line 221 are a marking cut directly in the specimen contact means, a marker capable of recognizing the exciting light or a light-collecting position and having a reflectivity. This also applies to the specimen recognition locus 222, but it is desirable to use a marker which does not influence fluorescent light.

The grip portion is not necessarily provided on the outer periphery.

What recognizes the detection-starting point is not limited to the line.

The detection locus may be spiral or oblique.

Further, the start point for the movement of the exciting light is not limited to the outer periphery.

Example 13 of the present invention will be described below.

In a microorganism counting process, first, 4',6-diamidino-2-phenylindole dihydrochloride which is a first compound and propidium iodide which is a second compound are brought in the form of respective solutions or in the form of a reagent mixture into contact with a specimen having cells or microorganisms deposited thereon. The 4',6-diamidino-2-phenylindole dihydrochloride is passed through cell membranes on the specimen and bonded to nucleic acid. The propidium iodide cannot be passed through living cell membranes because of its large molecular weight, and is bonded to nucleic acid in dead cells.

The 4',6-diamidino-2-phenylindole dihydrochloride bonded to nucleic acid in living and dead cells absorbs light having an exciting wavelength of 359 nm to emit fluorescent light having a wavelength of 461 nm, thereby coloring or dyeing the living and dead cells. The propidium iodide bonded to nucleic acid in dead cells absorbs light having an exciting wavelength of 535 nm to emit fluorescent light having a wavelength of 617 nm, thereby coloring or dyeing the dead cells. The colored cells are counted visually or by a counter, whereby the number of the living and dead cells and the number of the dead cells are confirmed.

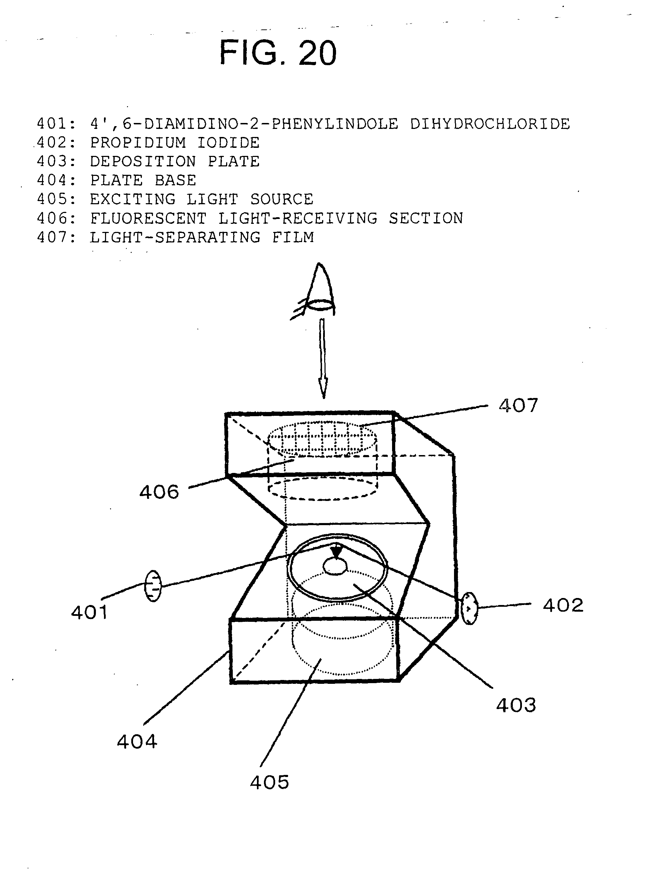

The microorganism counting process will be described with reference to Fig.20. In the microorganism counting process, 4',6-diamidino-2-phenylindole dihydrochloride 401 and propidium iodide 402 are used as detecting reagents, and an apparatus is used, which is comprised of a deposition plate 403 made of glass and having cells or microorganisms in a specimen deposited thereon, a plate base 404 on which the deposition plate 403 is placed, an exciting light source 405 mounted at a lower portion of the plate base 404 for applying exciting light to the deposition pate 403, a fluorescent light-receiving section 406 including a magnifying mirror for confirming fluorescent colors developed by cells or microorganisms in the specimen, and a light-separating film 407 mounted at an upper portion of the fluorescent light-receiving section 406 for limiting the wavelength of fluorescent light.

In the counting process, a detecting reagent mixture prepared by mixing 4',6-diamidino-2-phenylindole dihydrochloride 401 and propidium iodide 402 is used. A solution containing cells or microorganisms collected from a specimen is deposited or dropped onto the surface of the deposition plate 403; the deposition plate 403 is placed onto the plate base 404, and the detecting reagent mixture is dropped onto the surface of the deposition plate 403. The exciting light source 405 mounted at the lower portion of the deposition plate 403 is turned on, whereby the cells or microorganisms on the deposition plate 403 are stained or colored in a fluorescent manner, and fluorescent colors developed are magnified by the fluorescent light-receiving section 406. Further, by the light-separating film 407 transmits light having particular wavelengths, the cells or microorganisms emitting fluorescent rays are lighted individually. The cells or microorganisms lighted at the particular wavelengths are counted, whereby the number of living and dead cells and the number of dead cells are counted and thus, the number of existing living cells is calculated. In this manner, the number of the living cells and the number of the dead cells in the specimen can be confirmed.

The fluorescent light-receiving section 406 is provided with a lens of 200 to 1,000 magnifications.

Example 14 of the present invention will be described below.

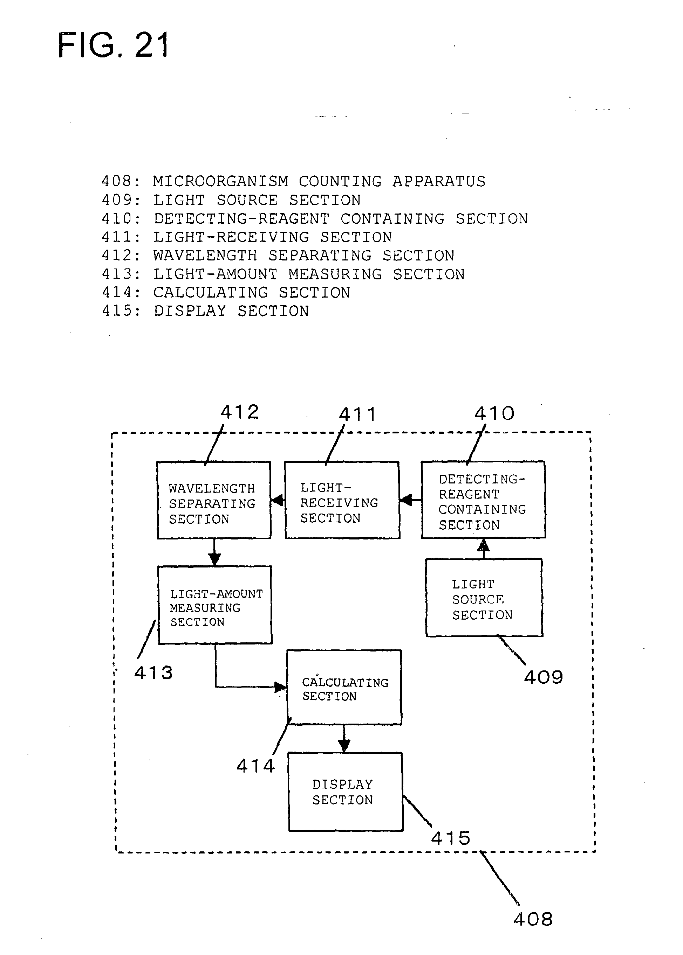

A block diagram of an apparatus for carrying out the counting automatically is shown in Fig. 21. As shown in Fig. 21, the microorganism counting apparatus 408 is comprised of a light source section 409 including a lamp for emitting exciting light, a detecting-reagent containing section 410 made of a glass, in which a solution containing 4',6-diamidino-2-phenylindole dihydrochloride 401 as a detecting reagent for staining or coloring living and dead cells of microorganisms collected from a specimen and propidium iodide 402 as a detecting reagent for staining or coloring dead cells of microorganisms is incorporated, a light-receiving section 411 for receiving colored light, a wavelength separating section 412 for separating light from the light-receiving section 411, a light-amount measuring section 413 for measuring an amount of light, a calculating section 414 for calculating the numbers of living cells and the dead cells, and a display section 415 for displaying the calculated numbers of the cells.

An example of use of the microorganism counting apparatus 408 will be described below.

In the use of the microorganism counting apparatus 408, 0. 5 grams of vegetables are dipped into 9.5 ml of a physiological saline to prepare a suspension. Then, 1 ml of the suspension, 4',6-diamidino-2-phenylindole dihydrochloride 401 and propidium iodide 402 are incorporated into the detecting-reagent containing section 410. After the incorporation, the detecting reagents and the suspension contained in the detecting-reagent containing section 410 are agitated automatically, whereby the detecting reagents are bonded to microorganism cells in the suspension. The lamp included in the light source section 409 for emitting exciting light is turned on to apply exciting light automatically to a specimen, whereby nucleic acid in living and dead cells bonded with the detecting reagents and nucleic acid in dead cells bonded with the detecting reagents emit rays, which are applied to the light-receiving section 411. The rays applied to the light-receiving section 411 are separated in the wavelength separating section 412 into rays having a fluorescent wavelength of 450 to 480 nm indicative of living and dead cells and rays having a fluorescent wavelength of 500 to 620 nm indicative of dead cells, and amounts of rays are converted into electric signals in the light-amount measuring section 413. The resulting electric signals are fed to the calculating section 414, where the numbers of cells are calculated from a calibration curve for number of cells and electric signals, and the number of the dead cells is subtracted from the number of the living and dead cells, thus calculating the number of the living cells. In this manner, the number of the living cells and the number of the dead cells in 1 ml of the suspension incorporated in the detecting-reagent containing section 410 can be displayed automatically on the display section 415.

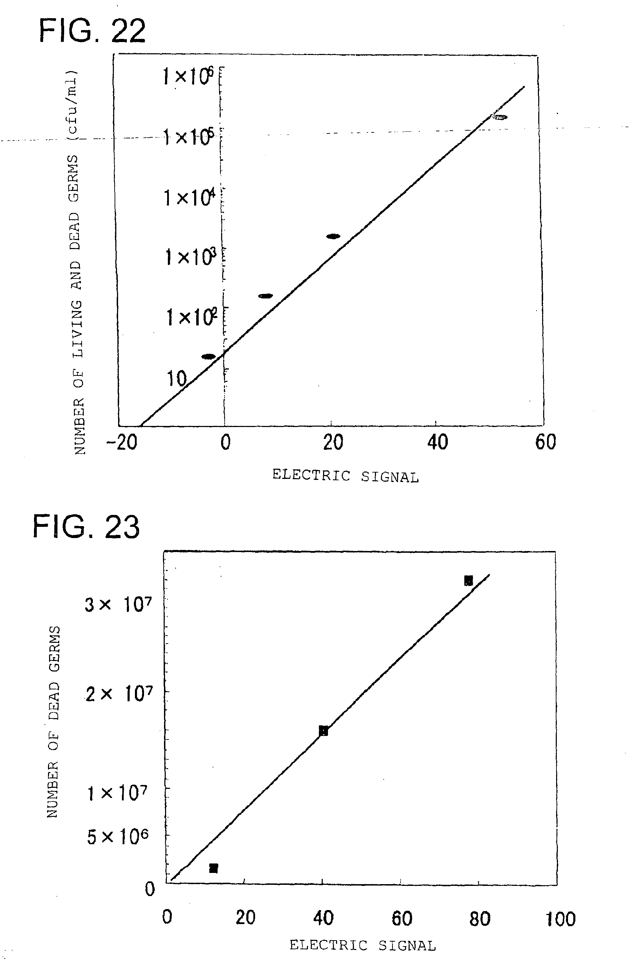

A graph of a calibration curve for living and dead cells and electric signal output is shown in Fig.22, and a graph of a calibration curve for dead cells and electric signal output is shown in Fig.23.

The amount of 4',6-diamidino-2-phenylindole dihydrochloride 401 dropped or added to the suspension is in a range of 10 n mole/ml (inclusive) to 1 µ mole/ml (inclusive) in terms of a final concentration with respect to the amount of a specimen, and an optimal concentration is 100 n mole/ml. The amount of propidium iodide 402 dropped or added to the suspension is in a range of 10 n mole/ml (inclusive) to 1 µ mole/ml (inclusive) in terms of a final concentration with respect to the amount of a specimen, and an optimal concentration is 100 n mole/ml.

The exciting light source 405 used is one adapted to emit light having an exciting wavelength in a range of 400 to 700 nm.

According to the present invention, the number of cells or microorganisms in a specimen can be detected visually or automatically, promptly and precisely by using the fluorescent coloration.

The deposition plate 403 made of a glass is used in Example 13, but a material such as an acrylic resin and a polymer film capable of transmitting exciting light may be used.

The amounts of rays has been converted into electric signals to count the number of cells in Examples 13 and 14, but a process which comprises picking up pictures to count the number of cells in terms of the amount of lighted pictures may be used.

The detecting-reagent containing section 410 made of a glass is used in Example 14 , but a material capable of transmitting exciting light and fluorescent light may be used.

Example 15 of the present invention will be described below.

In a microorganism counting process, first, a reagent mixture made by mixing 4',6-diamidino-2-phenylindole dihydrochloride as a first compound, propidium iodide as a second compound, 6-oarboxyfluoresoein diacetate as a third compound and 4-methylumbelliferyl-β-D-galaotoside as a fourth compound is brought into a specimen having cells or microorganisms deposited thereto. The 4',6-diamidino-2-phenylindole dihydrochloride bonded to nucleic acid in living and dead cells absorbs exciting light having an exciting wavelength of 359 nm to emit fluorescent light having a wavelength of 461 nm to color living and dead cells. The propidium iodide bonded to nucleic acid in dead cells absorbs exciting light having an exciting wavelength of 535 nm to emit fluorescent light having a wavelength of 617 nm to color only dead cells. The 6-carboxyfluorescein diacetate reacting with esterase in living cells is decomposed by the esterase to produce fluorescein, which absorbs exciting light having an exciting wavelength of 474 nm to emit fluorescent light having a wavelength of 541 nm to color only living cells. The 4-methylumbelliferyl-β-D-galaotoside reacts with β-galaotosidase produced as a metabolite from Coliform bacteria to produce 4-methylumbelliferone, which absorbs exciting light having an exciting wavelength of 394 nm to emit fluorescent light having a wavelength of 489 nm to color only Coliform bacteria. The number of living and dead cells and the number of dead cells are counted from the number of the colored cells and thus, the numbers of the living cells and the dead cells are calculated. Further, the presence or absence of living cells, and living Coliform bacteria as particular microorganisms can be confirmed.

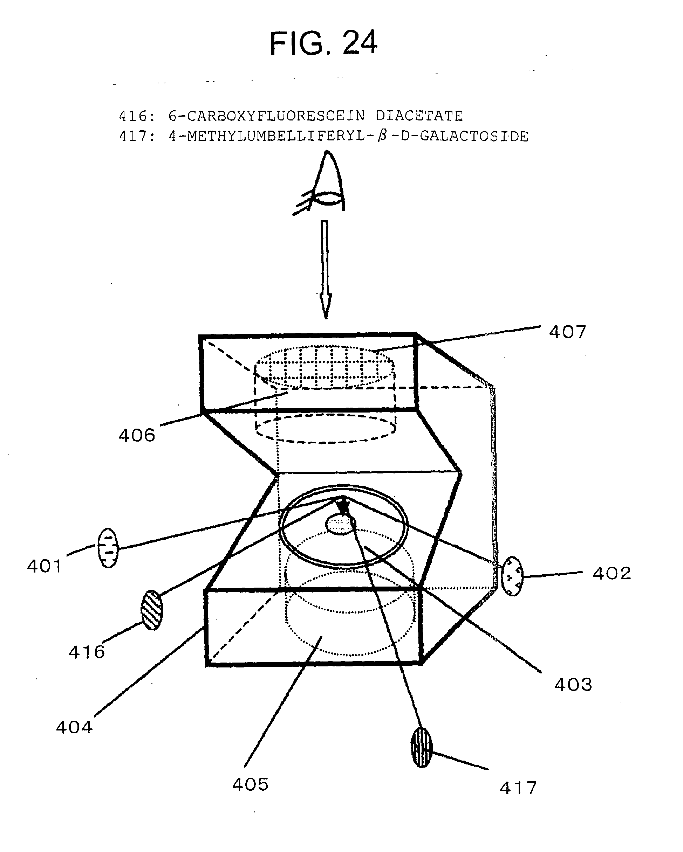

The microorganism counting process of the present invention shown in Fig.24 will be described below. In the microorganism counting process, 4',6-diamidino-2-phenylindole dihydrochloride 401, propidium iodide 402, 6-carboxyfluorescein diacetate 416 and 4-methylumbelliferyl-β-D-galaatoside 417 are used as detecting reagents, and an apparatus is used, which is comprised of a deposition plate 403 made of glass and having cells or microorganisms deposited thereon, a plate base 404 on which the deposition plate 403 is placed, an exciting light source 405 mounted at a lower portion of the plate base 404 for applying exciting light to the deposition pate 403, a fluorescent light-receiving section 406 mounted above the deposition plate 404 and including a magnifying mirror for catching colors developed by cells or microorganisms in the specimen, and a light-separating film 407 mounted at an upper portion of the fluorescent light-receiving section 406 for limiting the wavelength of fluorescent light.

In the counting process, a detecting reagent mixture prepared by mixing 4',6-diamidino-2-phenylindole dihydrochloride 401, propidium iodide 402, 6-carboxyfluorescein diacetate 416 and 4-methylumbelliferyl-β-D-galactoside 417 is used. The deposition plate 403 with a solution deposited thereon and containing cells or microorganisms collected from a specimen is placed onto the plate base 404, and the detecting reagent mixture is dropped onto the surface of the deposition plate 403. The exciting light source 405 mounted at the lower portion of the deposition plate 403 is turned on, whereby the cells or microorganisms on the deposition plate 403 are stained or colored in a fluorescent manner, and the fluorescent rays are magnified and transmitted by the fluorescent light-receiving section 406. Further, by the light-separating film 407 transmits light having particular wavelengths, the cells or microorganisms emitting fluorescent rays are lighted individually. Thus, the living and dead cells and the dead cells can be confirmed visually, and further, the presence or absence of highly active living cells and Coliform bacteria can be confirmed visually.

When the living and dead cells are counted, the light-separating film 407 capable of transmitting fluorescent light having a wavelength in a range of 450 to 480 nm is used. When the dead cells are counted, the light-separating film 407 capable of transmitting fluorescent light having a wavelength in a range of 500 to 620 nm is used. When the living cells are counted, the light-separating film 407 capable of transmitting fluorescent light having a wavelength in a range of 450 to 560 nm is used. When the Coliform bacteria are counted, the light-separating film 407 capable of transmitting fluorescent light having a wavelength in a range of 400 to 550 nm is used.

Example 16 of the present invention will be described below.

An apparatus for automatically counting microorganisms will be described with reference to Fig.21, because it has an arrangement similar to that shown in Fig.21. As shown in Fig. 21, the microorganism counting apparatus 408 is comprised of a light source section 409 including a lamp for emitting exciting light, a detecting-reagent containing section 410 made of a glass, in which a solution containing 4',6-diamidino-2-phenylindole dihydrochloride 401 as a detecting reagent for staining or coloring living and dead cells of microorganisms collected from a specimen and propidium iodide 402 as a detecting reagent for staining or coloring dead cells of microorganisms, 6-carboxyfluorescein diacetate 416 as a detecting reagent for staining or coloring living cells of microorganisms and 4-methylumbelliferyl-β-D-galaotoside 417 as a detecting reagent for staining or coloring Coliform bacteria is incorporated, a light-receiving section 411 for receiving colored light, a wavelength separating section 412 for separating light from the light-receiving section 411, a light-amount measuring section 413 for measuring an amount of light, a calculating section 414 for calculating the numbers of living cells and the dead cells or the number of Coliform bacteria, and a display section 415 for displaying the calculated numbers of the cells.

An example of use of the microorganism counting apparatus 408 will be described below.

In the use of the microorganism counting apparatus 408, 0. 5 grams of vegetables are dipped into 9.5 ml of a physiological saline to prepare a suspension. Then, 1 ml of the suspension, 4',6-diamidino-2-phenylindole dihydrochloride 401, propidium iodide 402, 6-carboxyfluoresoein diacetate 416 and 4-methylumbelliferyl-β-D-galactoside 417 are incorporated into the detecting-reagent containing section 410. After the incorporation, the detecting reagents and the suspension contained in the detecting-reagent containing section 410 are agitated automatically, whereby the detecting reagents are bonded to microorganism cells in the suspension. The lamp included in the light source section 409 for emitting exciting light is turned on to apply exciting light automatically to a specimen, whereby nucleic acid in living and dead cells bonded with the detecting reagents and nucleic acid in dead cells bonded with the detecting reagents are colored, and fluorescein and 4-methylubbelliferon are colored by the action of esterase in living cells and β-galactosidase produced as a metabolite from Coliform bacteria, thereby emitting rays to the light-receiving section 411. The rays applied to the light-receiving section 411 are separated in the wavelength separating section 412 into rays having a fluorescent wavelength of 461 nm indicative of living and dead cells, rays having a fluorescent wavelength of 617 nm indicative of dead cells, rays having a fluorescent wavelength of 541 nm indicative of living cells and rays having a fluorescent wavelength of 489 nm indicative of Coliform bacteria, and amounts of rays are converted into electric signals in the light-amount measuring section 413. The resulting electric signals are fed to the calculating section 414, where the numbers of cells are calculated from a calibration curve for number of cells and electric signals, and the number of the living cells is calculated from a difference between the number of the living and dead cells and the number of the dead cells. In this manner, the number of the living and dead cells, the number of the dead cells, the number of highly active living cells and the number of Coliform bacteria in 1 ml of the suspension incorporated in the detecting-reagent containing section 410 can be displayed automatically on the display section 415.

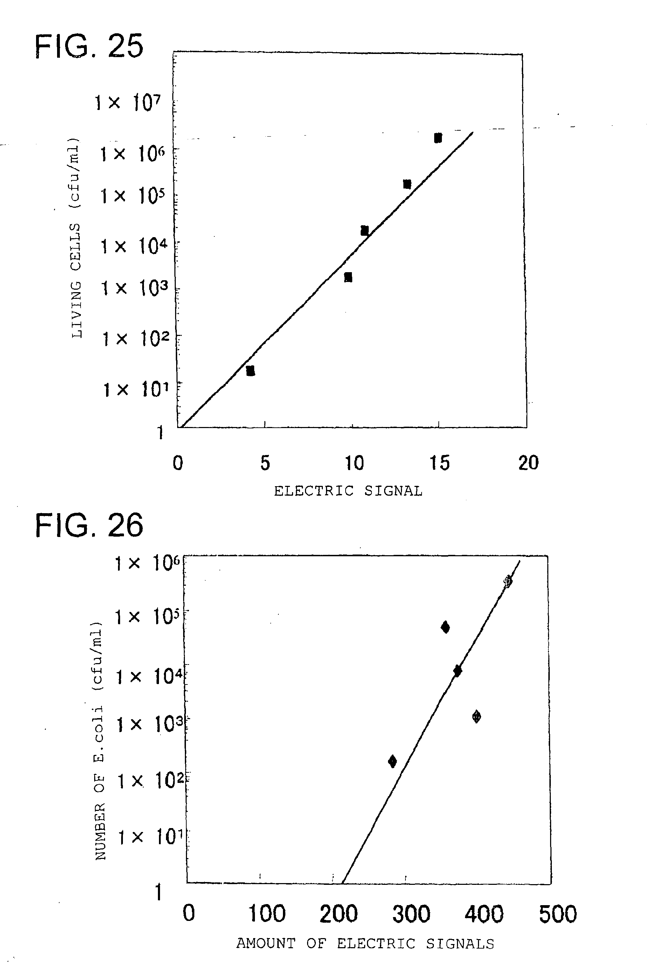

A graph of a calibration curve for highly active living cells and electric signal output is shown in Fig. 25, and a graph of a calibration curve for Coliform bacteria and electric signal output is shown in Fig.26.

According to the present invention, the number of living 6 cells and the number of dead cells can be counted by counting the number of living and dead cells and the number of dead cells by wavelengths of fluorescent colored rays, and can be detected promptly, simply and visually and further, the presence or absence of microorganisms such as Coliform bacteria and the number of highly active living cells can be confirmed.

### Industrial Applicability

As can be seen from the above-described Examples, according to the present invention, it is possible to provide a microorganism detecting kit, which includes a specimen contact means containing one or some of the following compounds incorporated therein: a first compound for staining living and dead cells, a second compound for staining dead cells at a wavelength different from the above-described staining, a third compound for staining living cells at a wavelength different from the above -described staining, and at least one or more fourth compounds for staining particular microorganisms at a wavelength different from the above-described staining by the reaction with a substance derived from particular microorganisms, and which has an effect that living and dead cells, dead cells and particular microorganisms can be monitored at a real time without being cultured.

In addition, it is possible to provide a microorganism detecting kit, which includes a specimen contact means containing the following compounds incorporated therein: one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms, and which has an effect that living and dead cells, living cells, dead cells and particular microorganisms can be detected, so that the kit can be also utilized to estimate an anti-fungal effect.

Further, it is possible to provide a microorganism detecting kit, which Includes a detecting-reagent containing section and a specimen contact means, the detecting-reagent containing section containing one or some of the following compounds are incorporated therein: a first compound for staining living and dead cells, a second compound for staining dead cells at a wavelength different from the above-described staining, a third compound for staining living cells at a wavelength different from the above-described staining, and at least one or more fourth compounds for staining particular microorganisms at a wavelength different from the above-described staining by the reaction with a substance derived from particular microorganisms, and which has an effect that the counting of the number of cells and the determination of the type of germs deposited to a specimen can be carried out promptly and simultaneously.

Yet further, it is possible to provide a microorganism detecting kit, which includes a detecting-reagent containing section and a specimen contact means, the detecting-reagent containing section containing the following compounds incorporated therein: one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms, and which has an effect that, when there are a large amount of cells or microorganisms deposited to a specimen, living and dead cells, living cells, dead cells and particular microorganisms can be detected, and the life and death of the particular microorganisms can be discriminated from each other, so that the kit can be utilized to estimate polluted foods.

Yet further, it is possible to provide a microorganism detecting kit wherein the staining or coloration is fluorescent, thereby enhancing the sensitiveness and providing an effect that it is possible to prevent the operator's mis-reoognition by preventing the interference of light by using the detecting reagents suited to emit different fluorescent rays.

Yet further, it is possible to provide a microorganism detecting kit wherein the first compound is one capable of being bonded to nucleic acid, thereby providing an effect that cells can be detected precisely to a one-cell level and hence, the number of living and dead cells can be counted.

Yet further, it is possible to provide a microorganism detecting kit wherein the second compound is one capable of being bonded to nucleic acid, thereby providing an effect that cells can be detected precisely to a one-cell level and hence, the number of dead cells can be counted.

Yet further, it is possible to provide a microorganism detecting kit wherein the third compound is one capable of developing a color by the reaction with a substance derived from microorganisms, thereby providing an effect that the amount of living microorganisms can be grasped, and the degree of pollution of living microorganisms can be grasped.

Yet further, it is possible to provide a microorganism detecting kit wherein the substance derived from microorganisms is an enzyme protein, thereby providing an effect that the number of living microorganisms can be grasped with a good sensitiveness, and the degree of pollution of living microorganisms can be grasped with a good sensitiveness.

Yet further, it is possible to provide a microorganism detecting kit wherein the substance derived from particular microorganisms is an enzyme protein, thereby providing an effect that the presence of microorganisms such as disease germs can be grasped with a further good sensitiveness and hence, the kit can be utilized to examine environments and to grasp promptly the polluted situation in a field such as a medical field.

Yet further, it is possible to provide a microorganism detecting kit wherein at least two or more of the first compound, second compound, third compound and fourth compound are contained in separated states, thereby providing an effect that even if the compounds are those suited to emit rays having closer wavelengths by the reaction, microorganisms can be detected easily visually.

Yet further, it is possible to provide a microorganism detecting kit wherein the specimen contact means contains a carrier for supporting at least one or more of the first compound, second compound, third compound and fourth compound, thereby providing an effect that the detecting kit can be preserved easily, and microorganisms can be detected with a further good sensitiveness by increasing the area of contact with a specimen.

Yet further, it is possible to provide a microorganism detecting kit wherein the detecting-reagent containing section contains a carrier for supporting at least one or more of the first compound, second compound, third compound and fourth compound, thereby providing an effect that the detecting kit can be preserved easily, and microorganisms can be detected with a further good sensitiveness by increasing the area of contact with a specimen. There is further provided an effect that even if the compounds are those suited to emit rays having closer wavelengths by the reaction, microorganisms can be detected easily visually.

Yet further, it is possible to provide a microorganism detecting kit wherein the specimen contact means is of a size equal to that of the light-reoeiving means for receiving rays emitted from the specimen contact means, thereby providing an effect that cells can be detected without need for scanning.

Yet further, it is possible to provide a microorganism detecting kit wherein the specimen contact means includes at least one or more of the first compound, second compound, third compound and fourth compound supported on its surface to be brought into contact with a specimen, thereby providing an effect that the amount of the compound(s) can be suppressed to the minimum.

Yet further, it is possible to provide a microorganism detecting kit wherein the specimen contact means contains a culture medium for culturing microorganisms, thereby providing an effect that it is possible to maintain the activity of microorganisms and to prevent a reduction in activity microorganisms and hence, to detect microorganisms at higher sensitiveness.

Yet further, it is possible to provide a microorganism detecting kit wherein the specimen contact means has a self-adherent property on its surface to be brought into contact with the specimen, thereby providing an effect that microorganisms existing in the specimen can be collected precisely and hence, the state of the specimen polluted by the microorganisms can be grasped reliably.

Yet further, it is possible to provide a microorganism detecting kit wherein the specimen contact means is provided with a grip portion, thereby providing an effect that it is possible to prevent an operator from touching a specimen or the specimen contact means brought into contact with the specimen.

Yet further, it is possible to provide a microorganism detecting kit wherein the microorganism detecting kit itself has a function to transmit only light having a particular wavelength, thereby providing an effect that it is unnecessary to specially separate rays and hence, the confirmation of colors developed can be simplified.

Yet further, it is possible to provide a microorganism detecting kit wherein the detecting-reagent containing section has a function to transmit only light having a particular wavelength, thereby providing an effect that it is unnecessary to specially separate rays and hence, the confirmation of colors developed can be simplified, and the switching placement of a means for separating light having another wavelength can be carried out easily.

Yet further, it is possible to provide a microorganism detecting kit further including a preservation reservoir in which even a liquid specimen can be stored at a given thickness, thereby providing an effect that even if the specimen is liquid, microorganisms contained in the liquid specimen can be retained in constant states in the preservation reservoir, and the thickness can be maintained constant, whereby an error due to a difference in thickness can be reduced.

In addition, as can be seen from the above-described Examples, according to the present invention, it is possible to provide a microorganism counting apparatus wherein among microorganisms existing in a specimen, the numbers of all germs, the number of living germs, the number of dead germs, the number of particular germs existing in the specimen or the presence or absence of germs can be detected using compounds excited by exciting light having different wavelengths to emit fluorescent rays having different wavelengths. Further, the extinction of the fluorescent rays emitted by virtue of the exciting light is inhibited to carry out the detection precisely, and it is possible to prevent overestimation due to the incorporation of foreign matters by providing threshold values, thereby maintaining a high sensitiveness.

Further, it is possible to provide a microorganism counting apparatus wherein detecting reagents are contained in the detecting-reagent containing section, and the detecting reagents are separated from the specimen contact means, whereby the specimen contact means can be deposited directly to a specimen, and when a subject of detection is a food, a medical appliance or the like, a sanitary state can be maintained by preventing the deposition of the detecting reagents. Thus, it is possible to bring the specimen contact means into direct contact to the specimen and hence to detect microorganisms at a high sensitiveness.

Further, it is possible to provide a microorganism counting apparatus having a high sensitiveness, wherein microorganisms are transferred from a specimen to the specimen contact means by a transfer means, whereby microorganisms contained in the specimen can be deposited to the specimen contact means with a good efficiency.

Yet further, it is possible to provide a microorganism counting apparatus wherein even when a specimen is liquid, microorganisms can be counted by using a preservation reservoir for preserving the liquid specimen, and further, it is possible to prevent the overestimation or underestimation due to diffusion of microorganisms collected from the specimen by preserving the liquid specimen in the preservation reservoir to provide a given thickness.

Yet further, it is possible to provide a microorganism counting apparatus wherein the staining or coloration is fluorescent, thereby providing an effect that the sensitiveness can be enhanced and further, a plurality of germs can be detected at one time by using detecting reagents suited to emit fluorescent rays having different wavelengths.

In addition, it is possible to provide a microorganism counting apparatus wherein the compound for detecting living germs is a substance reacting with a substance derived from microorganisms, whereby the activity of microorganisms can be estimated from a production amount of the substance derived from microorganisms. In this case, the number of colored microorganisms can be grasped by counting. Further, it is possible to provide a microorganism counting apparatus wherein compounds capable of being excited by light having a different wavelengths and bonded to nucleic acid are used, thereby providing an effect that nucleic acid retained in cells can be dyed, and cells can be detected precisely to a one-cell level.

Further, it is possible to provide a microorganism counting apparatus wherein particular microorganisms can be detected by utilizing a compound developing a color by a substance produced by particular microorganisms and further, the intended substance is an enzyme protein, thereby providing an effect that particular microorganisms can be detected at a high sensitiveness, and the activity of particular microorganisms can be also grasped.

Further, it is possible to provide a microorganism counting apparatus wherein the light-receiving means comprises at least one photoelectric converting element, and a collector lens for collecting rays emitted by virtue of exciting light is mounted in front of the photoelectric converting element(s), whereby a high detecting sensitiveness can be provided. Further, the linear arrangement of the photoelectric converting elements provides an effect that microorganisms existing on the specimen contact means can be detected by the unidirectional scanning, and the counting of microorganisms can be increased in speed.

Further, it is possible to provide a microorganism counting apparatus wherein the specimen contact means is of a disk-shape, whereby the driving of the specimen contact means in a rotational direction can be facilitated, thereby increasing the detection speed. Further, the distance of the specimen-detecting movement of at least one of the light source and the light-receiving means can be suppressed to the minimum. This provides an effect that it is possible to shorten the detecting time and to simplify the microorganism counting apparatus.

Yet further, it is possible to provide a microorganism counting apparatus wherein the very small area for application of exciting light emitted from the light source to the specimen is movable in a preset range of speed, thereby providing an effect that it is possible to prevent the slippage of the color developed, the persistence of vision and the afterglow, thereby preventing the overestimation or the underestimation.

Yet further, it is possible to provide a microorganism counting apparatus wherein at least one of the light emitted from the light source and the light emitted by the specimen contact means is collected using a reflecting plate, thereby providing an effect that the highly efficient application of light to the specimen contact means and the highly efficient detection in the light-receiving means are possible, thereby simplifying the arrangement of the apparatus.

Yet further, it is possible to provide a microorganism counting apparatus wherein a light-separating section capable of separating the wavelengths of light from the light source and a light-separating section capable of separating the wavelengths of fluorescent rays are mounted, thereby providing an effect that light having a target wavelength can be applied, and the emitted fluorescent rays of target wavelengths can be detected, thereby enabling the intended detection of a high sensitiveness.

Yet further, it is possible to provide a microorganism counting apparatus wherein the surface of the specimen contact means is recognized automatically, and the focus of the exciting light is adjusted, whereby the detecting position can be recognized, and the entire specimen contact means can be detected. Thus, it is possible to prevent the double detection or the omission of detection to avoid the overestimation or the underestimation of the specimen.

Yet further, it is possible to provide a microorganism counting apparatus wherein the exciting light from the light source is introduced utilizing an optical fiber, whereby the light source as a heat source can be placed at another location. Thus, it is possible to prevent the influence of heat to microorganisms collected on the specimen contact means, the associated protein and the like to avoid an increase in error. Further, even when the specimen contact means is of a complicated shape, light can be introduced in an amount constant at all times to the specimen contact means, and the more precise counting can be achieved.

Yet further, it is possible to provide a microorganism counting apparatus wherein a lens capable of transmitting ultraviolet rays is used as a collector means for light emitted from the light source, thereby providing an effect that light having a wavelength in an ultraviolet range can be introduced into the specimen contact means without reduction in amount of light, and the detectable compounds can be counted based on the wavelength in the ultraviolet range.

Yet further, it is possible to provide a microorganism counting apparatus wherein a means for indicating the measuring position is provided on the apparatus or on the specimen contact means, a detecting position can be recognized, whereby the mis-detection can be prevented, or the specimen-detecting speed can be regulated. Further, a magnetic means is utilized as the means for indicating the measuring position on a specimen contact means, thereby enabling the simplified and precise detection.

Yet further, it is possible to provide a microorganism counting apparatus wherein a means is provided for moving the measuring position on the specimen contact means from a current recognizing locus position to a next recognizing locus position with the distance kept constant. Thus, it is unnecessary to detect the entire specimen, and an increase in detecting speed can be achieved. In addition, it is possible to prevent the mis-recognition of microorganisms existing between the locus positions and to carry out the precise counting. Further, the distance between the locus positions is in a range of 10 to 50 µm, thereby providing an effect that the correlation to the prior art process can be provided.

Yet further, it is possible to provide a microorganism counting apparatus wherein fluorescent rays emitted from microorganisms contained in the specimen contact means are recognized as a binary punctual coordinates comprising "0" and "1". Thus, microorganisms can be counted easily and quickly, and objects recognized as foreign matters due to the positional relationship can be determined as being out of subject and eliminated, and those of microorganisms recognized as microorganisms, which have been recognized as adjacent microorganisms likewise due to the positional relationship, are recognized as one microorganisms, as compared with the agar culture medium diffusion process which is the prior art process, thereby providing an effect that the correlation to the prior art process can be provided.

Yet further, it is possible to provide a microorganism counting apparatus wherein in order to count microorganisms utilizing the emission of colored rays, the means adapted to receive the emitted rays by at least one light-receiving element received the rays within a given time and judged as microorganisms. Thus, the emitted rays can be recognized by the light-receiving element and detected within the given time, thereby inhibiting an error due to the extinction of luminaries to count the number of microorganisms precisely.

Yet further, it is possible to provide a microorganism counting apparatus wherein when the intensity of rays emitted and recognized is fallen within preset threshold values, the rays are judged as microorganisms. Thus, foreign matters and microorganisms can be distinguished from each other, whereby the number of microorganisms can be counted precisely.

Yet further, it is possible to provide a microorganism counting apparatus wherein when rays emitted from microorganisms recognized by a light-receiving element and rays entering a light-receiving element adjoining such light-receiving element have been recognized as microorganisms, these microorganisms are judged all together as one group of microorganisms, thereby maintaining the correlation to the culture process which is one of prior art processes, which is that when microorganisms are cultured, colonies are coalesced together with the growth of adjacent microorganisms and recognized as one colony.

Yet further, it is possible to provide a microorganism counting apparatus including a light source and a light-receiving means, so that cells are counted from a difference between wavelengths of rays emitted from a specimen and amounts of fluorescent colors developed. Thus, an artificial error can be minimized, and cells can be counted easily even by those not skilled in the art.

As can be seen from the above-described Examples, according to the present invention, it is possible to provide a microorganism counting process comprising the steps of bringing a first compound for staining living and dead cells and a second compound for staining dead cells at a wavelength different from the above-described staining into contact with a specimen, and detecting both of living cells and dead cells simultaneously from a difference between wavelengths and a difference between amounts of colors developed. Thus, the living and dead cells and the dead cells can be measured precisely, and the polluted situation of environments by cells can be grasped, thereby quickly taking a measure to prevent the pollution. In addition, the toxicity to cells, an effect of sterilization of microorganisms and the like can be confirmed.

Additionally, it is possible to provide a microorganism counting process comprising the steps of bringing one or more of a first compound for staining living and dead cells, a second compound for staining dead cells at a wavelength different from the above-described staining, and a third compound for staining living cells at a wavelength different from the above-described staining; and at least one or more fourth compounds for staining particular microorganisms at a wavelength different from the above-described staining by the reaction with a substance derived from particular microorganisms, into contact with a specimen, and detecting both or either one of living cells and dead cells, and particular microorganisms simultaneously from a difference between wavelengths and amounts of colors developed. Thus, the monitoring of environments such as the pollution of foods, water or the like can be carried out at a real time, and the process can be utilized not only in a medical field but also in a food sanitation field. In addition, the presence of particular microorganisms such as E.coli can be grasped promptly, thereby quickly preventing the influence to a market and a distribution due to the pollution of microorganisms.

In addition, it is possible to provide a microorganism counting process wherein the staining or coloration is fluorescent, thereby providing an effect that it is possible to prevent the mis-recognition from being provided by a measuring operator when light is received, and to count the number of cells precisely with a good accuracy.

Further, it is possible to provide a microorganism counting process wherein the first compound is a compound capable of being bonded to nucleic acid. This provides the following effect: Cells can be measured even in the presence of metabolites produced by cells and polluted substances. Therefore, living and dead cells even in a specimen such as food materials and sludge can be measured directly and hence, the examining time can be reduced, and the cells can be measured quickly.

Further, it is possible to provide a microorganism counting process wherein the second compound is a compound capable of being bonded to nucleic acid. This provides the following effect: Cells can be measured even in the presence of metabolites produced by cells and polluted substances. Therefore, living and dead cells even in a specimen such as food materials and sludge can be measured directly and hence, the examining time can be reduced and further, the cells can be measured quickly.

Further, it is possible to provide a microorganism counting process wherein the third compound is a compound for staining microorganisms in a specimen by the reaction with a substance derived from microorganisms, thereby providing an effect that the presence or absence of living microorganisms can be confirmed. In addition, it is possible to provide a microorganism counting process wherein the substance derived from microorganisms is an enzyme protein, thereby providing an effect that the presence or absence of living microorganisms can be judged with a higher sensitiveness.

Further, it is possible to provide a microorganism counting process wherein the substance derived from particular microorganisms is an enzyme protein, thereby providing an effect that the presence or absence of special microorganisms can be confirmed, and microorganisms can be monitored. In addition, a pretreatment is not required and further, the examining time can be shortened.

Further, it is possible to provide a microorganism counting process comprising the step of bringing a compound for staining particular microorganisms by the reaction with a substance derived from particular microorganisms into contact with a specimen, and detecting the particular microorganisms from a wavelength and an amount of color developed. This provides an effect that only the particular microorganisms can be counted quickly and hence, it is possible to grasp the polluted situation of foods and to previously prevent the incorporation of the particular microorganisms to a human body.

## Claims

1. A microorganism counting process comprising the steps of bringing 4', 6-diamidino-2-phenylindole dihydrochloride for staining living and dead cells and propidium iodide for staining dead cells at a wavelength different from said coloration into contact with a specimen, and detecting both of living cells and dead cells simultaneously from a difference between wavelengths and difference between amounts of colors developed.
